# EUROPÄISCHE PATENTANMELDUNG

(11) **EP 4 410 369 A2**
(43) Veröffentlichungstag der Anmeldung: **07.08.2024**
(21) Anmeldenummer: 24182989.4
(22) Anmeldetag: 25.09.2012
(51) Int. Cl.: A61P 11/02

(54) **Zusammensetzung für die nasale Applikation mit verbesserter Stabilität**

(30) Priorität: 30.12.2011 DE 102011122588; 20.03.2012 DE 102012005452
(62) Teilanmeldung aus: 14182601.6
(71) Anmelder: Maria Clementine Martin Klosterfrau Vertriebsgesellschaft mbH, 50670 Köln (DE)
(72) Erfinder: GREVE, Harald, 40545 Düsseldorf (DE)
(74) Vertreter: Strehlke, Ingo Kurt

(57) **Zusammenfassung**

Die Erfindung betrifft eine für die topische, insbesondere nasale, vorzugsweise intranasale Applikation bestimmte pharmazeutische Zusammensetzung mit verbesserter Stabilität sowie deren Verwendung und eine diese pharmazeutische Zusammensetzung enthaltende Applikationsvorrichtung.

## Beschreibung

Die vorliegende Erfindung betrifft das Gebiet der Behandlung von Rhinitiden.

Insbesondere betrifft die vorliegende Erfindung eine Zusammensetzung, insbesondere eine pharmazeutische Zusammensetzung, mit verbesserter Stabilität, welche sich vorzugsweise für die topische, insbesondere nasale, bevorzugt intranasale Applikation eignet, insbesondere für die Behandlung von Rhinitiden, und die Verwendung dieser Zusammensetzung sowie eine diese Zusammensetzung enthaltende Applikationsvorrichtung.

Unter einer Rhinitis (synonym gelegentlich auch als Nasenkatarrh, Schnupfen oder Koryza bezeichnet) versteht man im Rahmen der vorliegenden Erfindung insbesondere eine akute oder chronische Entzündung der Nasenschleimhäute, wobei die Rhinitis insbesondere infektiösen, allergischen oder pseudoallergischen Ursprungs sein kann. Am Häufigsten tritt eine Rhinitis im Rahmen einer sogenannten Erkältung auf.

Neben einer Unterscheidung von akuter Rhinitis einerseits und chronischer Rhinitis andererseits unterscheidet man auch verschiedene Formen der Rhinitis, beispielsweise die *Rhinitis acuta, Rhinitis atrophicans, Rhinitis allergica, Rhinitis hypertrophica, Rhinitis medicamentosa, Rhinitis pseudomembranacea, Rhinitis sicca* und *Rhinitis vasomotorica.*

Bei der sogenannten akuten Rhinitis (*Rhinitis acuta*), d. h. dem gewöhnlichen Schnupfen, handelt es sich in der Regel um einen im Allgemeinen harmlosen Infekt der Nasenschleimhäute und damit um eine infektiöse Rhinitis, welche durch eine Vielzahl von Viren (insbesondere Rhinoviren und/oder Adenoviren) ausgelöst werden kann; Hauptmerkmal einer akuten Rhinitis ist eine sogenannte laufende Nase und eine Verstopfung der Nase durch die Anschwellung der Schleimhäute.

Insgesamt sind mehr als 200 "Schnupfenviren" als mögliche Auslöser einer viralen Rhinitis bekannt, wie sie üblicherweise im Rahmen einer gewöhnlichen Erkältung auftreten kann. Im Rahmen einer Erkältung, welche im Allgemeinen mit einer Rhinitis beginnt, verschwindet aber die *Rhinitis acuta* im Allgemeinen. Gelegentlich kann es jedoch mitunter auch zu einer Chronifizierung kommen, welche oftmals mit einer Volumenzunahme der Schleimhäute unter anderem im Bereich der Nasenmuscheln mit Behinderung der Nasenatmung einhergeht.

Für weitergehende Einzelheiten zum Begriff der Rhinitis kann insbesondere verwiesen werden auf Pschyrembel, Medizinisches Wörterbuch, 257. Auflage, Seiten 1331/1332, insbesondere Stichwörter: "*Rhinitis*", "*Rhinitis allergica*", *"Rhinitis atrophicans", "Rhinitis hyperplastica", "Rhinitis pseudomembranacea*", "*Rhinitis sicca*" und "*Rhinitis vasomotorica*".

Da es also eine Vielzahl verschiedener Typen von Viren gibt, welche eine Rhinitis auslösen können, und da eine Vielzahl von Ursachen für das Auftreten einer Rhinitis existieren, können Rhinitiden, insbesondere akute Rhinitiden, im Allgemeinen nicht kausal, sondern nur symptomatisch, vorzugsweise topisch, behandelt werden, zumeist unter Anwendung von Sympathomimetika (synonym auch als sogenannte sogenannter "Abschweller" oder "Dekongestiva" bezeichnet), vorzugweise alpha-Sympathomimetika, wie Xylometazolin und Oxymetazolin oder deren physiologisch verträgliche Salze.

Diese Sympathomimetika führen aufgrund ihrer vasokonstriktorischen Eigenschaften nach lokaler bzw. topischer Anwendung in der Nase zwar zu einer Nasenschleimhautabschwellung, bewirken jedoch bei wiederholten Anwendung oftmals eine Austrocknung der Nasenschleimhäute, einhergehend mit entzündlichen Irritationen der Nasenschleimhäute - was nicht selten zu einer erhöhten Infektionsgefahr führt, da die Nasenschleimhäute im ausgetrockneten und entzündeten Zustand nicht mehr ihre Schutz- und Filterfunktionen im vollen Umfang aufrechterhalten können und folglich Krankheitserreger ungehindert in die Atemwege gelangen können.

Zudem sind diese Sympathomimetika, insbesondere Xylometazolin und Oxymetazolin sowie deren physiologisch verträglichen Salze, nicht unbegrenzt in wässriger Lösung lagerbar, so dass unerwünscht und unkontrolliert Abbauprodukte dieser Wirkstoffe entstehen können, welche dann einer Anwendung entsprechender wässriger Lösungen dieser Wirkstoffe entgegenstehen können.

So ist es beispielsweise für das alpha-Sympathomimetikum Xylometazolinhydrochlorid bekannt, dass es in wässriger Lösung hydrolytisch gespalten werden kann. Wie die nachfolgende Reaktionsgleichung zeigt, wird Xylometazolinhydrochlorid (1) in wässriger Lösung hydrolytisch unter Öffnung des Imidazolinrings gespalten, so dass als Abbauprodukt das Amid (2) gebildet wird, welches in der Monographie des Europäischen Arzneibuchs (Ph. Eur.) auch als sogenannte "Verunreinigung A" bezeichnet wird:

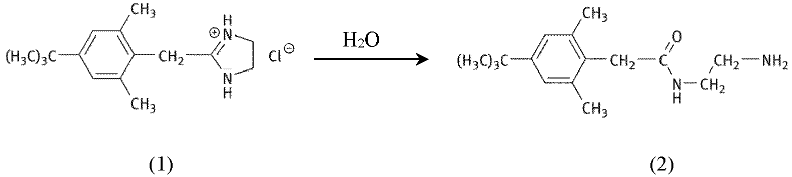

Für das Oxymetazolinhydrochlorid gilt ein analoger Mechanismus, welcher durch die folgende Reaktionsgleichung wiedergegeben werden kann, wobei das Oxymetazolinhydrochlorid (3) hydrolytisch zu dem Amid (4) als Abbauprodukt umgewandelt wird:

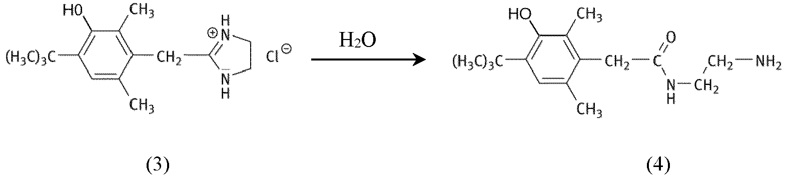

Um einem unerwünschten Abbau der Abschweller auf Sympathomimetikumbasis, insbesondere auf Basis von Xylometazolin und/oder Oxymetazolin sowie deren Salzen, entgegenzuwirken, wurden im Stand der Technik verschiedene Maßnahmen ergriffen, welche sich aber als unzulänglich erwiesen haben bzw. mit unerwünschten Nachteilen verbunden sind:
So führt die Zugabe externer Stabilisatoren oftmals zu einer nur unzureichenden Stabilisierung dieser Wirkstoffe. Eine Zugabe komplexierender Salze, insbesondere von Zinksalzen, führt zwar zu einer gewissen Stabilitätsverbesserung (vgl. DE 103 37 186 A1 und WO 2005/018601 A1), führt jedoch bei nasaler Applikation zu einer unerwünschten Reizung der Nasenschleimhäute infolge der Zinksalze, einhergehend mit entzündlichen Irritationen.

Daher können die im Stand der Technik aufgezeigten Versuche bzw. Lösungsansätze zur Stabilisierung der vorgenannten sympathomimetikabasierten Wirkstoffe in wässrigen Systemen nur als praktizierter Kompromiss in Ermangelung einer besseren Alternative angesehen werden. Zudem berücksichtigen zu vorgenannten Lösungsansätze nicht den austrocknenden Effekt dieser Wirkstoffe in Bezug auf die Nasenschleimhäute.

Um andererseits einer Austrocknung der Nasenschleimhäute durch die Anwendung von alpha-Sympathomimetika entgegenzuwirken, wurde im Stand der Technik die kombinierte Verabreichung von Pantothenol und/oder Pantothensäure (vgl. die zu derselben Patentfamilie gehörenden Druckschriften DE 195 41 919 A1, DE 195 49 421 A1 und EP 1 663 141 B1) bzw. Hyaluronsäure (vgl. DE 103 56 248 A1) vorgeschlagen, da diese Wirkstoffe (d. h. Pantothenol bzw. Pantothensäure bzw. Hyaluronsäure und deren jeweils physiologisch verträgliche Salze) einer Austrocknung der Nasenschleimhäute durch die Anwendung der alpha-Sympathomimetika in effizienter Weise entgegenwirken. In diesem Zusammenhang hat sich insbesondere die Anwendung von Pantothenol bzw. Pantothensäure bewährt.

Insbesondere der Wirkstoff Pantothenol (synonym auch als "Dexpanthenol" bezeichnet) kann jedoch in wässriger Lösung hydrolytisch gemäß der nachfolgenden Reaktionsgleichung gespalten werden, wobei sich aus dem Wirkstoff Panthenol (3) in saurer Lösung - neben dem Ammoniumsalz des 3-Aminopropanols (7) - das Lacton D-Pantolacton (6) als Abbauprodukt bildet, während in alkalischer Lösung - neben 3-Aminopropanol (5) - das Salz der Pantosäure (4) gebildet wird:

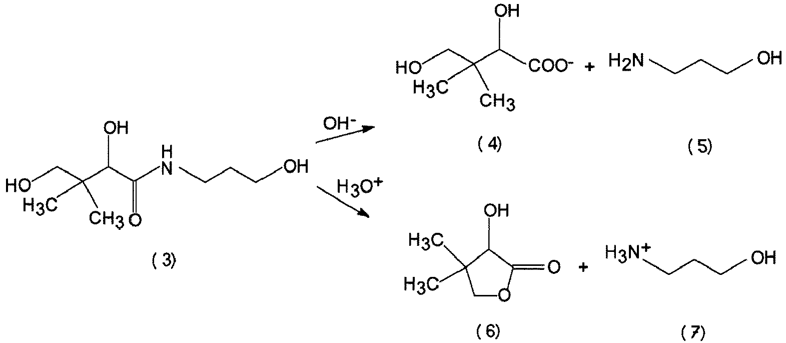

In gemeinsamer wässriger Lösung von alpha-Sympathomimetika, insbesondere auf Basis von Xylometazolin und/oder Oxymetazolin, einerseits und Panthenol bzw. Pantothensäure andererseits ist eine Stabilisierung der einzelnen Inhaltsstoffe besonders diffizil, da die einzelnen Wirkstoffe (d. h. Xylometazolin bzw. Oxymetazolin einerseits und Panthenol bzw. Dexpanthenol andererseits) in unterschiedlichen pH-Bereichen ihr Stabilitätsoptimum aufweisen.

Das der vorliegenden Erfindung zugrundeliegende Problem besteht daher in der Bereitstellung einer für die topische, insbesondere nasale, bevorzugt intranasale Applikation, insbesondere für die Behandlung von Rhinitiden, geeigneten Zusammensetzung, insbesondere pharmazeutischen Zusammensetzung, welche die zuvor geschilderten Nachteile des Standes der Technik zumindest weitgehend vermeidet oder aber wenigstens abschwächt, insbesondere eine verbesserte Stabilität (z. B. Lagerstabilität) aufweist. Insbesondere soll eine solche Zusammensetzung auch nach längerer Lagerung keine bzw. keine signifikanten Mengen an Abbauprodukten der Inhaltsstoffe, insbesondere keine signifikanten Mengen an Abbauprodukten des Xylometazolins und/oder Oxymetazolins und des Panthenols bzw. der Pantothensäure, aufweisen.

Zur Lösung des zuvor geschilderten Problems schlägt die vorliegende Erfindung - gemäß einem ersten Aspekt der vorliegenden Erfindung - eine Zusammensetzung, insbesondere eine pharmazeutische Zusammensetzung, nach Anspruch 1 vor; weitere, insbesondere vorteilhafte Ausgestaltungen der erfindungsgemäßen Zusammensetzung sind Gegenstand der diesbezüglichen Unteransprüche.

Weiterhin betrifft die vorliegende Erfindung - gemäß einem zweiten Aspekt der vorliegenden Erfindung - eine die erfindungsgemäße Zusammensetzung enthaltende Applikationsvorrichtung gemäß dem betreffenden Vorrichtungsanspruch.

Schließlich betrifft die vorliegende Erfindung - gemäß einem weiteren und dritten Aspekt der vorliegenden Erfindung - die erfindungsgemäße Verwendung der Zusammensetzung nach der vorliegenden Erfindung, wie sie in den entsprechenden Verwendungsansprüchen definiert ist.

Es versteht sich bei den nachfolgenden Ausführungen von selbst, dass Ausgestaltungen, Ausführungsformen, Vorteile und dergleichen, welche nachfolgend zu Zwecken der Vermeidung von Wiederholungen nur zu einem Erfindungsaspekt ausgeführt sind, selbstverständlich auch in Bezug auf die übrigen Erfindungsaspekte entsprechend gelten, ohne dass dies einer gesonderten Erwähnung bedarf.

Bei allen nachstehend genannten relativen bzw. prozentualen gewichtsbezogenen Angaben, insbesondere Mengenangaben, ist weiterhin zu beachten, dass diese im Rahmen der vorliegenden Erfindung vom Fachmann derart auszuwählen sind, dass sie sich in der Summe unter Einbeziehung aller Komponenten bzw. Inhaltstoffe, insbesondere wie nachfolgend definiert, stets zu 100 % bzw. 100 Gew.-% ergänzen bzw. addieren; dies versteht sich aber für den Fachmann von selbst.

Im Übrigen gilt, dass der Fachmann - anwendungsbezogen oder einzelfallbedingt - von den nachfolgend angeführten Gewichts-, Mengen- und Bereichsangaben abweichen kann, ohne dass er den Rahmen der vorliegenden Erfindung verlässt.

Zudem gilt, dass alle im Folgenden genannten Werte- bzw. Parameterangaben oder dergleichen grundsätzlich mit genormten bzw. standardisierten oder explizit angegebenen Bestimmungsverfahren oder andernfalls mit dem Fachmann auf diesem Gebiet an sich geläufigen Bestimmungs- bzw. Messmethoden ermittelt bzw. bestimmt werden können.

Dies vorausgeschickt, wird die vorliegende Erfindung nunmehr nachfolgend im Detail erläutert.

Gegenstand der vorliegenden Erfindung - gemäß einem **ersten** Aspekt der vorliegenden Erfindung - ist somit eine Zusammensetzung, insbesondere eine pharmazeutische Zusammensetzung, welche vorzugsweise für die topische, insbesondere nasale, bevorzugt intranasale Applikation, insbesondere für die Behandlung von Rhinitiden, geeignet ist,
wobei die Zusammensetzung
a) mindestens ein imidazolinbasiertes alpha-Sympathomimetikum oder dessen physiologisch unbedenkliches Salz;
   und
b) (b1) Pantothenol (Dexpanthenol) oder dessen physiologisch unbedenkliche Ester und/oder
(b2) Pantothensäure oder deren physiologisch unbedenkliche Salze enthält, wobei der pH-Wert der Zusammensetzung im Bereich von 5,0 bis 6,2 eingestellt und/oder konstantgehalten ist.

Denn, wie die Anmelderin vollkommen überraschend herausgefunden hat, gelingt es bei Einstellung bzw. Konstanthaltung dieses sehr engen pH-Bereichs von 5,0 bis 6,2, einerseits das imidazolinbasierte alpha-Sympathomimetikum und andererseits auch das Pantothenol (Dexpanthenol) bzw. die Pantothensäure in effizienter Weise zu stabilisieren, ohne dass eine nennenswerte Hydrolyse oder ein anderweitiger Abbau dieser beiden Wirkstoffe bzw. Wirkstoffkomponenten eintritt.

Mit anderen Worten lässt sich unter Einhaltung des vorgenannten, sehr engen pH-Regimes von 5,0 bis 6,2 eine Stabilisierung beider Wirkstoffe a) und b) auch in gemeinsamer wässriger Lösung erreichen, insbesondere ohne den zusätzlichen Einsatz von Stabilisatoren oder Konservierungsmittel.

Dies ist umso überraschender, als in diesem leicht sauren pH-Bereich von 5,0 bis 6,2 sowohl ein hydrolytischer Abbau des alpha-Sympathomimetikums als auch des Pantothenols bzw. der Pantothensäure zu erwarten gewesen wäre, da dieses Phänomen in Lösungen der Einzelwirkstoffe beobachtet wird. Überraschenderweise tritt ein hydrolytischer Abbau der beiden Wirkstoffkomponenten a) und b) jedoch nicht ein, sofern einerseits die beiden vorgenannten Wirkstoffkomponenten a) und b) in gemeinsamer Lösung vorliegen und andererseits dieser gezielt und eng ausgewählte pH-Wertbereich von 5,0 bis 6,2 konstantgehalten bzw. eingestellt wird. Das Auffinden dieses Effekts, insbesondere im Hinblick auf die Stabilisierung der Zusammensetzung, ist vollkommen überraschend und war angesichts des zuvor geschilderten Standes der Technik in dieser Weise nicht zu erwarten. Hierauf wird im Nachfolgenden noch im Detail eingegangen werden.

Erfindungsgemäß erfolgt im Rahmen der vorliegenden Erfindung die Bestimmung bzw. Messung des pH-Wertes mit dem Fachmann an sich bekannten bzw. geläufigen oder aber unter standardisierten Methoden, insbesondere bei 20 °C und Atmosphärendruck (1.013,25 mbar). Bekanntermaßen bezeichnet der sogenannte pH-Wert eine dimensionslose Maßzahl, welche nach DIN 19260: 1971-03 als der negative dekadische Logarithmus der Wasserstoffionen-Aktivität definiert wird. Im Rahmen der vorliegenden Erfindung bezieht sich die Angabe des pH-Werts insbesondere auf die Bestimmungsmethode gemäß DIN 19266: 2000-01, welche die ältere DIN 19266: 1979-08 ersetzt. Für weitergehende Einzelheiten zum Begriff des pH-Werts und seiner Messung und Bestimmung kann insbesondere verwiesen werden auf RÖMPP Lexikon Chemie, 10. Auflage, Georg-Thieme-Verlag, Stuttgart/New York, Band 4, 1998, Seiten 3230 bis 3232, Stichwort: "*pH*", sowie auf die dort referierte Literatur, wobei die gesamte vorgenannte Literaturstelle mit der dort referierten Literatur hiermit durch Bezugnahme eingeschlossen sind.

Was die Komponente a) bzw. das imidazolinbasierte alpha-Sympathomimetikum der erfindungsgemäßen Zusammensetzung anbelangt, so ist diese Komponente a) gemäß einer besonderen Ausführungsform der vorliegenden Erfindung insbesondere ausgewählt aus Xylometazolin oder Oxymetazolin, insbesondere in Form von deren physiologisch unbedenklichen Salzen, besonders bevorzugt in Form von deren Hydrochloridsalzen.

Gemäß einer bevorzugten Ausführungsform der vorliegenden Erfindung ist die Komponente a) bzw. das imidazolinbasierte alpha-Sympathomimetikum ausgewählt aus Xylometazolin, insbesondere in Form von dessen physiologisch unbedenklichen Salzen, besonders bevorzugt in Form von dessen Hydrochloridsalz (Xylometazolinhydrochlorid).

Was die Komponente b) der erfindungsgemäßen Zusammensetzung anbelangt, so ist diese insbesondere aus Pantothenol (Dexpanthenol) oder dessen physiologisch unbedenklichen Estern ausgewählt. Vorzugsweise ist die Komponente b) der erfindungsgemäßen Zusammensetzung Pantothenol (Dexpanthenol).

Gemäß einer besonderen Ausführungsform enthält die erfindungsgemäße Zusammensetzung
a) mindestens ein imidazolinbasiertes alpha-Sympathomimetikum aus der Gruppe von Xylometazolin und/oder Oxymetazolin oder deren physiologisch unbedenklichen Salzen, besonders bevorzugt in Form von deren Hydrochloridsalzen; und
b) Pantothenol (Dexpanthenol) oder dessen physiologisch unbedenkliche Ester.

Gemäß einer erfindungsgemäß bevorzugten Ausführungsform enthält die erfindungsgemäße Zusammensetzung
a) Xylometazolin oder deren physiologisch unbedenkliche Salze, besonders bevorzugt in Form von dessen Hydrochloridsalz (Xylometazolinhydrochlorid); und
b) Pantothenol (Dexpanthenol) oder dessen physiologisch unbedenkliche Ester, vorzugsweise Pantothenol (Dexpanthenol).

Wie nachfolgend noch geschildert, bewirkt bei topischer, insbesondere nasaler, bevorzugt intranasaler Applikation der erfindungsgemäßen Zusammensetzung, insbesondere bei der Behandlung von Rhinitiden, die Wirkkomponente a) aufgrund ihrer vasokonstriktorischen Eigenschaften eine Abschwellung der Nasenschleimhäute, während die Wirkkomponente b) eine Austrocknung und entzündlichen Irritation der Schleimhäute, insbesondere infolge der Anwendung des alpha-Sympathomimetikums, entgegenwirkt.

Die Menge an Komponente a) in der erfindungsgemäßen Zusammensetzung kann in weiten Bereichen variieren. Um eine ausreichende Wirkung der Komponente a) in der erfindungsgemäßen Zusammensetzung zu gewährleisten, ist es vorteilhaft, wenn die erfindungsgemäße Zusammensetzung die Komponente a), bezogen auf die erfindungsgemäße Zusammensetzung, in einer Menge von 0,001 bis 2 Gew.-%, insbesondere 0,005 bis 1,5 Gew.-%, vorzugsweise 0,01 bis 1,2 Gew.-%, bevorzugt 0,02 bis 1,0 Gew.-%, besonders bevorzugt 0,03 bis 0,5 Gew.-%, ganz besonders bevorzugt 0,04 bis 0,2 Gew.-%, enthält. Dennoch kann der Fachmann anwendungsbezogen oder einzelfallbedingt von den vorgenannten Mengenangaben abweichen, ohne dass er den Rahmen der vorliegenden Erfindung verlässt.

Gleichermaßen kann auch die Menge an der Komponente b) in der erfindungsgemäßen Zusammensetzung in weiten Bereichen variieren. Um eine ausreichende Wirkung durch die Komponente b) zu gewährleisten, ist es jedoch vorteilhaft, wenn die erfindungsgemäße Zusammensetzung die Komponente b), bezogen auf die erfindungsgemäße Zusammensetzung, in einer Menge von 0,01 bis 10 Gew.-%, insbesondere 0,1 bis 9 Gew.-%, vorzugsweise 0,5 bis 8 Gew.-%, bevorzugt 1 bis 7 Gew.-%, besonders bevorzugt 2 bis 6 Gew.-%, ganz besonders bevorzugt 3 bis 6 Gew.-%, enthält. Dennoch kann der Fachmann anwendungsbezogen oder einzelfallbedingt von den vorgenannten Mengenangaben abweichen, ohne dass er den Rahmen der vorliegenden Erfindung verlässt.

Für eine ausreichende Wirkung einerseits und eine zuverlässige Stabilisierung der beiden Komponenten a) und b) andererseits ist auch das Mengenverhältnis der Komponente a) zu der Komponente b) von Bedeutung. Insbesondere ist es in diesem Zusammenhang von Vorteil, wenn die erfindungsgemäße Zusammensetzung die Komponenten a) und b) in einem Mengenverhältnis von Komponente a) zu Komponente b) im Bereich von 1 : 10 bis 1 : 1.000, insbesondere 1 : 15 bis 1 : 500, vorzugsweise 1 : 20 bis 1 : 250, bevorzugt 1 : 25 bis 1 : 200, besonders bevorzugt 1 :30 bis 1 : 175, ganz besonders bevorzugt 1 : 40 bis 1 : 150, noch mehr bevorzugt 1 : 45 bis 1 : 125, enthält. Auf diese Weise werden Wirkeffizienz und Stabilität, insbesondere Lagerstabilität, in gleicher Weise gewährleistet. Dennoch ist es nicht ausgeschlossen, dass der Fachmann einzelfallbedingt oder anwendungsbezogen von den vorgenannten Wertebereichen abweichen kann, ohne dass er den Rahmen der vorliegenden Erfindung verlässt.

Im Allgemeinen liegt die erfindungsgemäße Zusammensetzung als wässrige Zusammensetzung vor. Insbesondere ist also die erfindungsgemäße Zusammensetzung wässrig basiert bzw. liegt die erfindungsgemäße Zusammensetzung wässrig formuliert vor, insbesondere in Form einer wässrigen Lösung oder wässrigen Solubilisierung.

Bevorzugterweise liegt somit die erfindungsgemäße Zusammensetzung als wässriges System, insbesondere als wässriges Einphasensystem, vorzugsweise als wässrige Lösung oder wässrige Solubilisierung, vor. Dabei weist die erfindungsgemäße Zusammensetzung somit einen Exzipienten oder Träger auf Wasserbasis auf.

Im Allgemeinen liegt die erfindungsgemäße Zusammensetzung als klare, farblose wässrige Lösung vor. Dies hat zum einen den Vorteil einer vereinfachten Applikation. Zum anderen lassen sich an einer klaren, farblosen wässrigen Lösung Veränderungen ohne Weiteres wahrnehmen.

Wie zuvor dargelegt, gelingt die Stabilisierung der erfindungsgemäßen Zusammensetzung, insbesondere der Wirkstoffe a) und b) der erfindungsgemäßen Zusammensetzung, durch Einstellung und/oder Konstanthaltung des pH-Werts der erfindungsgemäßen Zusammensetzung im Bereich von 5,0 bis 6,2. Besonders gute Ergebnisse, insbesondere im Hinblick auf die Stabilität, werden erhalten, wenn der pH-Wert der erfindungsgemäßen Zusammensetzung im Bereich von 5,0 bis 6,0, insbesondere im Bereich von 5,1 bis 6,0, vorzugsweise im Bereich von 5,2 bis 5,9, eingestellt und/oder konstantgehalten wird.

Üblicherweise erfolgt die Einstellung und/oder Konstanthaltung des pH-Werts der erfindungsgemäßen Zusammensetzung mittels mindestens eines chemischen Puffersystems, insbesondere mit Hilfe von Puffersalz(en).

Zum Begriff des chemischen Puffers der Puffersystems kann insbesondere verwiesen werden auf RÖMPP Lexikon Chemie, 10. Auflage, Georg-Thieme-Verlag, Stuttgart/New York, Band 5, 1998, Seiten 3618/3619, Stichwort: "*Puffer*", sowie auf die dort referierte Literatur.

Mit anderen Worten enthält die erfindungsgemäße Zusammensetzung also mindestens ein chemisches Puffersystem, insbesondere Puffersalz(e).

In diesem Zusammenhang hat es sich insbesondere bewährt, wenn zur Einstellung und/oder Konstanthaltung des pH-Werts der erfindungsgemäßen Zusammensetzung ein Dihydrogenphosphat/Monohydrogenposphat-Puffersystem verwendet wird, welches auch als "H₂PO₄⁻/HPO₄²⁻-Puffer(system)" bzw. "Phosphatpuffer(system)" bezeichnet wird; erfindungsgemäß besonders bevorzugt ist ein Alkalidihydrogenphosphat/Alkalimonohydrogenposphat-Puffersystem.

Denn, wie die Stabilitätsuntersuchungen der Anmelderin in überraschender Weise gezeigt haben, bewirkt nur ein solches Phosphatpuffersystem - gegenüber anderen möglichen einsetzbaren Puffersystemen - die angestrebte und zuverlässige Langzeitstabilisierung der erfindungsgemäßen Zusammensetzung auch über mehr als zwei Jahre. Mit anderen Puffersystemen dagegen, welche sich im vergleichbaren pH-Wertbereich einsetzen lassen, wie z. B. einem Kohlensäure/Bicarbonat-Puffersystem, einem Kohlensäure/Silikat-Puffersystem, einem Essigsäure/Acetat-Puffersystem, einem Citronensäure/Citrat-Puffersystem oder dergleichen, lassen sich derart gute Stabilitätsergebnisse nicht bzw. nicht immer zuverlässig erhalten. Ohne sich auf eine bestimmte Theorie festlegen zu wollen, lässt sich dieser Effekt des erfindungsgemäß eingesetzten Phosphatpuffersystems möglicherweise auf sekundäre Effekte, wie Komplexierungsreaktionen oder dergleichen, zurückführen.

Im Hinblick auf die angestrebte Langzeitstabilität bzw. Langzeitstabilisierung der erfindungsgemäßen Zusammensetzung hat es sich besonders bewährt, wenn das erfindungsgemäß zur Einstellung bzw. Konstanthaltung des pH-Werts verwendete Dihydrogenphosphat/Monohydrogenposphat-Puffersystem mit einem Dihydrogenphosphat/Monohydrogenposphat-Molverhältnis von größer 5 : 1, insbesondere im Bereich von 5 : 1 bis 110 : 1, besonders bevorzugt im Bereich von 6 : 1 bis 105 : 1, ganz besonders bevorzugt im Bereich von 7 : 1 bis 100 : 1, eingesetzt wird. Mit anderen Worten enthält die erfindungsgemäße Zusammensetzung aus den vorgenannten Gründen das Dihydrogenphosphat/Monohydrogenposphat-Puffersystem bevorzugterweise mit einem Dihydrogenphosphat/Monohydrogenposphat-Molverhältnis von größer 5 : 1, insbesondere im Bereich von 5 : 1 bis 110 : 1, besonders bevorzugt im Bereich von 6 : 1 bis 105 : 1, ganz besonders bevorzugt im Bereich von 7 : 1 bis 100 : 1.

Weiterhin kann es erfindungsgemäß vorgesehen sein, dass die erfindungsgemäße Zusammensetzung als weiteren Wirk- bzw. Inhaltsstoff c) mindestens ein Konservierungsmittel und/oder Desinfektionsmittel enthält. Das optional vorhandene Konservierungsmittel und/oder Desinfektionsmittel kann dabei insbesondere ausgewählt sein aus der Gruppe von (i) Alkylbenzyldimethylammoniumchloriden, insbesondere C₈-C₁₈-Alkylbenzyldimethylammoniumchloriden, und Mischungen von verschiedenen Alkylbenzyldimethylammoniumchloriden, vorzugsweise Benzalkoniumchlorid, (ii) para-Hydroxybenzoesäureestern und Mischungen von verschiedenen para-Hydroxybenzoesäureestern, vorzugsweise Nipa-Estern, (iii) Chlorhexidin sowie Kombinationen der vorgenannten Verbindungen. Besonders bevorzugt wird als Konservierungsmittel und/oder Desinfektionsmittel, sofern vorhanden, Benzalkoniumchlorid eingesetzt; bei Benzalkoniumchlorid handelt es sich um ein Gemisch aus Alkylbenzyldimethylammoniumchloriden, deren Alkylteil aus C₈-C₁₈-Ketten besteht, wobei Benzalkoniumchlorid insbesondere für seine desinfizierende und konservierende Wirkung bekannt ist. Sofern die Zusammensetzung ein Konservierungsmittel und/oder Desinfektionsmittel enthält, sollte die Menge an Konservierungsmittel und/oder Desinfektionsmittel, bezogen auf die erfindungsgemäße Zusammensetzung, 0,001 bis 10 Gew.-%, insbesondere 0,005 bis 5 Gew.-%, vorzugsweise 0,01 bis 2 Gew.-%, bevorzugt 0,01 bis 1 Gew.-%, besonders bevorzugt 0,01 bis 0,5 Gew.-%, betragen.

Im Rahmen der vorliegenden Erfindung konnte die Anmelderin vollkommen unerwartet herausfinden, dass die Verwendung eines Konservierungsmittels und/oder Desinfektionsmittels, insbesondere Benzalkoniumchlorid, zu einer zusätzlichen Stabilisierung der Wirkstoffkomponenten a) und b) führt. Darüber hinaus hat die Verwendung eines Konservierungsmittels bzw. Desinfektionsmittels auch anwendungsbezogene Vorteile, da der desinfizierende Effekt des Konservierungs- bzw. Desinfektionsmittels die Wirkung der Wirkstoffkomponenten a) und b) unterstützt und darüber hinaus einer Keimbildung in der erfindungsgemäßen Zusammensetzung in effizienter Weise entgegengewirkt wird.

Weiterhin kann es vorgesehen sein, dass die erfindungsgemäße Zusammensetzung als weiteren Wirk- bzw. Inhaltsstoff d) mindestens ein vorzugsweise saures Glykosaminoglykan oder dessen physiologisch unbedenkliche Salze oder Derivate, insbesondere Hyaluronsäure oder deren physiologisch unbedenkliche Salze, enthält, insbesondere in einer Menge von 0,0001 bis 10 Gew.-%, insbesondere 0,001 bis 5 Gew.-%, vorzugsweise 0,01 bis 2 Gew.-%, bezogen auf die erfindungsgemäße Zusammensetzung. Wie die Anmelderin überraschenderweise herausgefunden hat, können Glykosaminoglykane, insbesondere Hyaluronsäure bzw. deren Salze, die Wirkung der übrigen Wirk-bzw. Inhaltsstoffe, insbesondere des Panthenols bzw. der Pantothensäure, unterstützen. Insbesondere können die Glykosaminoglykane, vorzugsweise die Hyaluronsäure, förderlich in Bezug auf die Linderung von Infektionen und Entzündungen der Nasenschleimhaut wirken und darüber hinaus eine Beschleunigung der Regeneration von entzündetem Gewebe bewirken.

Weiterhin kann es erfindungsgemäß vorgesehen sein, dass die erfindungsgemäße Zusammensetzung als weiteren Wirk- bzw. Inhaltsstoff e) Ectoin oder mindestens ein Ectoinderivat, insbesondere ein Hydroxyectoin, enthält, insbesondere in einer Menge von 0,0001 bis 10 Gew.-%, insbesondere 0,001 bis 5 Gew.-%, vorzugsweise 0,01 bis 2 Gew.-%, bezogen auf die Zusammensetzung. Wie die Anmelderin überraschend herausgefunden hat, ist die Verwendung von Ectoin bzw. von Ectoinderivaten im Hinblick auf eine Beschleunigung der Regeneration entzündeter Nasenschleimhaut von Vorteil.

Des Weiteren kann es erfindungsgemäße vorgesehen sein, dass die erfindungsgemäße Zusammensetzung als weiteren Wirk- bzw. Inhaltsstoff f) Natriumchlorid enthält, insbesondere in einer Menge von 0,001 bis 5 Gew.-%, insbesondere 0,01 bis 2 Gew.-%, vorzugsweise 0,1 bis 1 Gew.-%, bezogen auf die Zusammensetzung. Natriumchlorid bewirkt insbesondere eine Befeuchtung der Nasenschleimhäute und somit eine Beschleunigung der Regeneration entzündeter Nasenschleimhäute.

Gleichermaßen kann es vorgesehen sein, dass die erfindungsgemäße Zusammensetzung mindestens einen weiteren Inhaltsstoff aufweist. Dieser weitere Inhaltsstoff kann insbesondere ausgewählt sein aus der Gruppe von Verarbeitungshilfsstoffen, Stabilisatoren, Emulgatoren, Antioxidantien, Feuchthaltemitteln, Verdickungsmitteln, Antiseptika, Farbstoffen, Aromastoffen, Riechstoffen, Duftstoffen, Streckmitteln, Bindemitteln, Netzmitteln, Vitaminen, Spurenelementen, Mineralstoffen, Mikronährstoffen und/oder ätherischen Ölen sowie deren Kombinationen.

Des Weiteren ist die Osmolalität der erfindungsgemäßen Zusammensetzung von Bedeutung. Die Osmolalität der erfindungsgemäßen Zusammensetzung kann in weiten Bereichen variieren. Um eine gute Verträglichkeit der erfindungsgemäßen Zusammensetzung in Bezug auf die Nasenschleimhaut zu gewährleisten, ist es von Vorteil, wenn die Zusammensetzung nach der vorliegenden Erfindung eine Osmolalität im Bereich von 300 bis 600 mosm/kg, insbesondere im Bereich von 310 bis 550 mosm/kg, vorzugsweise im Bereich von 300 bis 525 mosm/kg, bevorzugt im Bereich von 325 bis 510 mosm/kg, besonders bevorzugt im Bereich von 350 bis 500 mosm/kg, aufweist. Auf diese Weise ist bei topischer Anwendung der erfindungsgemäßen Zusammensetzung auf die Nasenschleimhäute eine gute Verträglichkeit gewährleistet.

Für eine gute Handhabbarkeit der erfindungsgemäßen Zusammensetzung ist es weiterhin von Vorteil, wenn die erfindungsgemäße Zusammensetzung bei einer Temperatur von 20 °C und bei einem Druck von 1.013,25 mbar eine relative Dichte, bezogen auf reines Wasser, im Bereich von 1,001 bis 1,2, insbesondere im Bereich von 1,005 bis 1,15, vorzugsweise im Bereich von 1,005 bis 1,105, aufweist.

Wie zuvor dargelegt, besitzt aufgrund der erfindungsgemäßen Maßnahmen, wie sie zuvor geschildert worden sind, die erfindungsgemäße Zusammensetzung eine ausgezeichnete Stabilität bzw. Langzeitstabilität, insbesondere Lagerstabilität. Insbesondere ist die erfindungsgemäße Zusammensetzung bei Temperaturen im Bereich von 20 °C bis 50 °C, bei einem Druck von 1.013,25 mbar und bei einer relativen Luftfeuchtigkeit im Bereich von 50 % bis 90 % mindestens 6 Monate, insbesondere mindestens 12 Monate, vorzugsweise mindestens 24 Monate, bevorzugt mindestens 36 Monate, stabil, insbesondere lagerstabil.

Die Stabilität der erfindungsgemäßen Zusammensetzung lässt sich durch das Vorhandensein bzw. Gehalt der Abbauprodukte der Wirkstoffe a) und b) bestimmen und charakterisieren. Wie eingangs geschildert, ist das charakteristische Abbauprodukt von Xylometazolin bzw. Xylometazolinhydrochlorid die sogenannte Verunreinigung A. Wie eingangs gleichermaßen geschildert, sind die typischen Abbauprodukte des Wirkstoffs b), insbesondere von Pantothenol, das Aminopropanol bzw. dessen Ammoniumsalz und D-Pantolacton.

In diesem Zusammenhang enthält die erfindungsgemäße Zusammensetzung einen Gehalt an Abbauprodukt(en) des Wirkstoffs a), insbesondere Verunreinigung A, von höchstens 5 Gew.-%, insbesondere von höchstens 3 Gew.-%, vorzugsweise von höchstens 2 Gew.-%, besonders bevorzugt von höchstens 1 Gew.-%, bezogen auf den Wirkstoff a); dieser allenfalls äußerst geringe Gehalt an Abbauprodukt(en) des Wirkstoffs a) wird auch nach Lagerung der erfindungsgemäßen Zusammensetzung bei Temperaturen im Bereich von 20 °C bis 50 °C, bei einem Druck von 1.013,25 mbar und bei einer relativen Luftfeuchtigkeit im Bereich von 50 % bis 90 % von mindestens 6 Monaten, insbesondere mindestens 12 Monaten, vorzugsweise mindestens 24 Monaten, bevorzugt mindestens 36 Monaten, noch eingehalten.

Weiterhin weist in diesem Zusammenhang die erfindungsgemäße Zusammensetzung einen Gehalt an Abbauprodukt(en) des Wirkstoffs b), insbesondere Aminopropanol und/oder D-Pantolacton, von jeweils höchstens 5 Gew.-%, insbesondere von jeweils höchstens 3 Gew.-%, vorzugsweise von jeweils höchstens 2 Gew.-%, besonders bevorzugt von jeweils höchstens 1 Gew.-%, bezogen auf den Wirkstoff b), auf; dieser allenfalls äußerst geringe Gehalt an Abbauprodukt(en) des Wirkstoffs b) wird auch nach Lagerung der Zusammensetzung bei Temperaturen im Bereich von 20 °C bis 50 °C, bei einem Druck von 1.013,25 mbar und bei einer relativen Luftfeuchtigkeit im Bereich von 50 % bis 90 % von mindestens 6 Monaten, insbesondere mindestens 12 Monaten, vorzugsweise mindestens 24 Monaten, bevorzugt mindestens 36 Monaten, noch eingehalten.

Insbesondere betrifft die vorliegende Erfindung eine wie zuvor geschilderte Zusammensetzung zur Verwendung bei der prophylaktischen und/oder kurativen topischen Behandlung von Rhinitiden, insbesondere *Rhinitis acuta.*

Des Weiteren betrifft die vorliegende Erfindung - gemäß einem **zweiten** Aspekt der vorliegenden Erfindung - eine Applikationsvorrichtung, welche eine wie zuvor beschriebene Zusammensetzung nach der vorliegenden Erfindung enthält, wobei die erfindungsgemäße Applikationsvorrichtung insbesondere für die topische, insbesondere nasale, vorzugsweise intranasale Applikation geeignet ist und bevorzugt in Form eines Behältnisses mit Tropf- oder Sprüheinrichtung vorliegt.

Erfindungsgemäß bevorzugt ist es, wenn die erfindungsgemäße Applikationsvorrichtung eine Sprüheinrichtung zur vorzugsweise gleichförmigen Ausbringung der Zusammensetzung nach der vorliegenden Erfindung in einer Menge pro Sprühstoß im Bereich von 25 µl bis 300 µl, insbesondere im Bereich von 50 µl bis 200 µl, vorzugsweise im Bereich von 75 µl bis 125 µl, aufweist.

Dabei ist es insbesondere vorgesehen, dass die erfindungsgemäße Applikationsvorrichtung ein Behältnis bzw. einen Vorratsbehälter mit einem Volumen im Bereich von 5 ml bis 100 ml, insbesondere 10 ml bis 50 ml, aufweist. Dieses Behältnis bzw. dieser Vorratsbehälter dient dann zur Aufnahme der erfindungsgemäßen Zusammensetzung.

Für weitergehende Einzelheiten zu der erfindungsgemäßen Applikationsvorrichtung kann auf die vorangehenden Ausführungen zu der erfindungsgemäßen Zusammensetzung verwiesen werden, welche im Hinblick auf die erfindungsgemäße Applikationsvorrichtung entsprechend gelten.

Weiterer Gegenstand der vorliegenden Erfindung - gemäß einem **dritten** Aspekt der vorliegenden Erfindung - ist die Verwendung einer wie zuvor beschriebenen Zusammensetzung nach der vorliegenden Erfindung zur prophylaktischen und/oder kurativen topischen Behandlung von Rhinitiden aller Art, insbesondere *Rhinitis acuta,* bzw. die Verwendung einer wie zuvor beschriebenen Zusammensetzung nach der vorliegenden Erfindung zur Herstellung eines Arzneimittels zur prophylaktischen und/oder kurativen topischen Behandlung von Rhinitiden aller Art, insbesondere *Rhinitis acuta.*

Insbesondere kann die erfindungsgemäße Zusammensetzung zur Behandlung von Rhinitiden aller Art, insbesondere *Rhinitis acuta, Rhinitis allergica, Rhinitis atrophicans, Rhinitis hyperplastica* oder *hypertrophicans, Rhinitis mutilans, Rhinitis nervosa* oder *vasomotorica* oder *Rhinitis pseudomembranacea,* vorzugsweise *Rhinitis acuta,* eingesetzt werden.

Wie zuvor beschrieben, kann im Rahmen der erfindungsgemäßen Verwendung die erfindungsgemäße Zusammensetzung, wie zuvor beschrieben, topisch, insbesondere nasal, vorzugsweise intranasal, appliziert werden. Dabei kann insbesondere die erfindungsgemäße Applikationseinrichtung, wie sie zuvor beschrieben worden ist, zur Anwendung kommen.

Für weitergehende Einzelheiten zu der erfindungsgemäßen Verwendung kann auf die vorangehenden Ausführungen zu den übrigen Erfindungsaspekten verwiesen werden, welche in Bezug auf die erfindungsgemäße Verwendung entsprechend gelten.

Weitere Ausgestaltungen, Abwandlungen und Variationen sowie Vorteile der vorliegenden Erfindung sind für den Fachmann beim Lesen der Beschreibung ohne Weiteres erkennbar und realisierbar, ohne dass er dabei den Rahmen der vorliegenden Erfindung verlässt.

Die folgenden Ausführungsbeispiele dienen lediglich der Veranschaulichung der vorliegenden Erfindung, ohne die vorliegende Erfindung jedoch hierauf zu beschränken.

### Ausführungsbeispiele:

### 1. Herstellungsbeispiele:

### Allgemeine Herstellvorschrift

Zur Herstellung von jeweils 1.000 g einer klaren wässrigen Lösung der erfindungsgemäßen Zusammensetzung wird in dem Fachmann an sich bekannter Weise vorgegangen: Zunächst wird bei Raumtemperatur und Umgebungsdruck in einem entsprechenden Glasgefäß mit Rühreinrichtung eine definierte Menge an gereinigtem Wasser (z. B. 300 ml bis 600 ml) vorgelegt und nachfolgend mit einer Lösung des gewählten Puffersystems auf einen vorgegeben pH-Wert im Bereich zwischen 5,2 und 5,9 (z. B. pH-Wert von ca. 5,5) unter anschließender Kontrolle des erreichten pH-Werts eingestellt. Anschließend wird hierzu die nachfolgend in den Rezepturbeispielen spezifizierte Dexpanthenolmenge hinzugegeben, und das Ganze wird dann durch gründliches Rühren klar gelöst. Der Lösung wird dann gegebenenfalls ein Konservierungs- bzw. Desinfektionsmittel, insbesondere Benzalkoniumchlorid, bevorzugt in Form einer wässrigen Lösung, zugesetzt und gleichfalls unter Rühren gelöst. Nachfolgend wird die gewünschte Xylometazolin- bzw. Oxymetazolinhydrochloridmenge zugesetzt, mit weiterem Wasser zum Endgewicht von 1.000 g aufgefüllt und homogen gerührt. Gegebenenfalls wird die Lösung über neutrale Cellulosefilter filtriert. Abschließend wird der pH-Wert noch einmal kontrolliert. Auch die übrigen entsprechenden Spezifikationen der Lösung werden auf die eingestellten bzw. vorgewählten Wertebereiche hin überprüft (z. B. Osmolalität: 400 bis 490 mosm/kg; relative Dichte bei 20 °C: 1,01 bis 1,05; mikrobiologische Reinheit und Sterilität; Ausschluss von Verunreinigungen, insbesondere Abbauprodukten der Inhalts- und Wirkstoffe). Ein Teil der erhaltenen Lösung wird schließlich in Enghalsflaschen aus Braunglas zu 10 ml oder 20 ml abgefüllt, welche wahlweise mit einer Tropfpipette oder einer Sprühdosierpumpe ausgerüstet werden können; 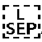 zur bestimmungsgemäßen Anwendung können mehrmals täglich ein bis drei Tropfen bzw. ein bis zwei Sprühstöße in jedes Nasenloch gegeben und aufgezogen werden. Ein anderer Teil der erhaltenen Lösung wird für die nachfolgend beschriebenen Stabilitätsuntersuchungen verwendet.

Nach dieser allgemeinen Herstellvorschrift werden die nachfolgend spezifizierten Rezepturen hergestellt.

### Rezeptur 1 (Angabe pro 10 g Zusammensetzung, zur Anwendung bei Erwachsenen):

| **Inhaltsstoff** | **Menge/mg** | **Qualität** |
|---|---|---|
| Xylometazolinhydrochlorid | 10,00 | Ph. Eur. |
| Dexpanthenol | 500,00 | Ph. Eur. |
| Puffersystem: | | Ph. Eur. |
| Kaliumdihydrogenphosphat / | 85,30 | |
| Natriummonohydrogenphosphat (Dodekahydrat) | 2,70 | |
| Gereinigtes Wasser | 9.402,00 | Ph. Eur. |

### Rezeptur 2 (Angabe pro 10 g Zusammensetzung, zur Anwendung bei Erwachsenen):

| **Inhaltsstoff** | **Menge/mg** | **Qualität** |
|---|---|---|
| Oxymetazolinhydrochlorid | 10,00 | Ph. Eur. |
| Dexpanthenol | 500,00 | Ph. Eur. |
| Puffersystem: | | Ph. Eur. |
| Kaliumdihydrogenphosphat / | 85,30 | |
| Natriummonohydrogenphosphat (Dodekahydrat) | 2,70 | |
| Gereinigtes Wasser | 9.402,00 | Ph. Eur. |

### Rezeptur 3 (Angabe pro 10 g Zusammensetzung, zur Anwendung bei Kindern):

| **Inhaltsstoff** | **Menge/mg** | **Qualität** |
|---|---|---|
| Xylometazolinhydrochlorid | 5,00 | Ph. Eur. |
| Dexpanthenol | 500,00 | Ph. Eur. |
| Puffersystem: | | Ph. Eur. |
| Kaliumdihydrogenphosphat / | 85,30 | |
| Natriummonohydrogenphosphat (Dodekahydrat) | 2,70 | |
| Gereinigtes Wasser | 9.407,00 | Ph. Eur. |

### Rezeptur 4 (Angabe pro 10 g Zusammensetzung, zur Anwendung bei Kindern):

| **Inhaltsstoff** | **Menge/mg** | **Qualität** |
|---|---|---|
| Oxymetazolinhydrochlorid | 5,00 | Ph. Eur. |
| Dexpanthenol | 500,00 | Ph. Eur. |
| Puffersystem: | | Ph. Eur. |
| Kaliumdihydrogenphosphat / | 85,30 | |
| Natriummonohydrogenphosphat (Dodekahydrat) | 2,70 | |
| Gereinigtes Wasser | 9.407,00 | Ph. Eur. |

### Rezeptur 5 (Angabe pro 10 g Zusammensetzung, zur Anwendung bei Erwachsenen):

| **Inhaltsstoff** | **Menge/mg** | **Qualität** |
|---|---|---|
| Xylometazolinhydrochlorid | 10,00 | Ph. Eur. |
| Dexpanthenol | 500,00 | Ph. Eur. |
| Puffersystem: | | Ph. Eur. |
| Kaliumdihydrogenphosphat / | 85,30 | |
| Natriummonohydrogenphosphat (Dodekahydrat) | 2,70 | |
| Konservierungs-/Desinfektionsmittel: | 4,00 | Ph. Eur. |
| Benzalkoniumchlorid (als 50 %-ige Lösung) | | |
| Gereinigtes Wasser | 9.398,00 | Ph. Eur. |

### Rezeptur 6 (Angabe pro 10 g Zusammensetzung, zur Anwendung bei Erwachsenen):

| **Inhaltsstoff** | **Menge/mg** | **Qualität** |
|---|---|---|
| Oxymetazolinhydrochlorid | 10,00 | Ph. Eur. |
| Dexpanthenol | 500,00 | Ph. Eur. |
| Puffersystem: | | Ph. Eur. |
| Kaliumdihydrogenphosphat / | 85,30 | |
| Natriummonohydrogenphosphat (Dodekahydrat) | 2,70 | |
| Konservierungs-/Desinfektionsmittel: | 4,00 | Ph. Eur. |
| Benzalkoniumchlorid (als 50 %-ige Lösung) | | |
| Gereinigtes Wasser | 9.398,00 | Ph. Eur. |

### Rezeptur 7 (Angabe pro 10 g Zusammensetzung, zur Anwendung bei Kindern):

| **Inhaltsstoff** | **Menge/mg** | **Qualität** |
|---|---|---|
| Xylometazolinhydrochlorid | 5,00 | Ph. Eur. |
| Dexpanthenol | 500,00 | Ph. Eur. |
| Puffersystem: | | Ph. Eur. |
| Kaliumdihydrogenphosphat / | 85,30 | |
| Natriummonohydrogenphosphat (Dodekahydrat) | 2,70 | |
| Konservierungs-/Desinfektionsmittel: | 4,00 | Ph. Eur. |
| Benzalkoniumchlorid (als 50 %-ige Lösung) | | |
| Gereinigtes Wasser | 9.403,00 | Ph. Eur. |

### Rezeptur 8 (Angabe pro 10 g Zusammensetzung, zur Anwendung bei Kindern):

| **Inhaltsstoff** | **Menge/mg** | **Qualität** |
|---|---|---|
| Oxymetazolinhydrochlorid | 5,00 | Ph. Eur. |
| Dexpanthenol | 500,00 | Ph. Eur. |
| Puffersystem: | | Ph. Eur. |
| Kaliumdihydrogenphosphat / | 85,30 | |
| Natriummonohydrogenphosphat (Dodekahydrat) | 2,70 | |
| Konservierungs-/Desinfektionsmittel: | 4,00 | Ph. Eur. |
| Benzalkoniumchlorid (als 50 %-ige Lösung) | | |
| Gereinigtes Wasser | 9.403,00 | Ph. Eur. |

### Rezeptur 9 (Angabe pro 10 g Zusammensetzung, zur Anwendung bei Erwachsenen):

| **Inhaltsstoff** | **Menge/mg** | **Qualität** |
|---|---|---|
| Xylometazolinhydrochlorid | 10,00 | Ph. Eur. |
| Dexpanthenol | 500,00 | Ph. Eur. |
| Puffersystem: | | Ph. Eur. |
| Kaliumdihydrogenphosphat / | 85,30 | |
| Natriummonohydrogenphosphat (Dodekahydrat) | 2,70 | |
| Konservierungs-/Desinfektionsmittel: | 4,00 | Ph. Eur. |
| Benzalkoniumchlorid (als 50 %-ige Lösung) | | |
| Hyaluronsäure (Natriumsalz) | 50,00 | |
| Natriumchlorid | 90,00 | |
| Hydroxyectoin | 50,00 | |
| Gereinigtes Wasser | 9.208,00 | Ph. Eur. |

### Rezeptur 10 (Angabe pro 10 g Zusammensetzung, zur Anwendung bei Kindern):

| **Inhaltsstoff** | **Menge/mg** | **Qualität** |
|---|---|---|
| Xylometazolinhydrochlorid | 5,00 | Ph. Eur. |
| Dexpanthenol | 500,00 | Ph. Eur. |
| Puffersystem: | | Ph. Eur. |
| Kaliumdihydrogenphosphat / | 85,30 | |
| Natriummonohydrogenphosphat (Dodekahydrat) | 2,70 | |
| Konservierungs-/Desinfektionsmittel: | 4,00 | Ph. Eur. |
| Benzalkoniumchlorid (als 50 %-ige Lösung) | | |
| Hyaluronsäure (Natriumsalz) | 50,00 | |
| Natriumchlorid | 90,00 | |
| Hydroxyectoin | 50,00 | |
| Gereinigtes Wasser | 9.213,00 | Ph. Eur. |

### 2. Anwendungsbeobachtungen und klinische Untersuchungen:

Je 20 Probanden eines aus 100 Patienten bestehenden Probandenkollektivs im Alter von 28 bis 79 Jahren (56 männlich, 44 weiblich), welche an akuten Rhinitiden litten, wurden mit den für die Erwachsenentherapie bestimmten Rezepturen 1 bzw. 2 bzw. 5 bzw. 6 bzw. 9 für die Dauer der Erkrankung (im Allgemeinen 3 bis 10 Tage) behandelt; die Rezepturen wurden mehrmals täglich als Spray topisch appliziert. Der vasokonstriktorische Effekt von Xylometazolinhydrochlorid bzw. Oxymetazolinhydrochlorid konnte, bezogen auf die Verbesserung der Symptomatik der Nasenschwellung und Behinderung der Nasenatmung, bei allen Patienten signifikant aufgezeigt werden. Aufgrund des Vorhandenseins der Dexpanthenolkomponente sowie gegebenenfalls weiterer Komponenten (z. B. Hyaluronsäure bzw. Hydroxyectoin bzw. Natriumchlorid) wurden keinerlei Reizwirkungen der Nasenschleimhäute infolge des Xylometazolinhydrochlorids bzw. Oxymetazolinhydrochlorids beobachtet. Insbesondere wurden auch keine Irritationen der Nasenschleimhäute infolge eines sogenannten "Rebound-Effektes", wie er bei Einsatz von Xylometazolinhydrochlorid bzw. Oxymetazolinhydrochlorid als alleinigem Wirksubstanz (d. h. ohne das Vorhandensein von Dexpanthenol) nach wiederholter Anwendung üblicherweise auftritt, beobachtet.

In analoger Weise und mit vergleichbarem Behandlungserfolg wurden je 5 Probanden eines aus 25 Patienten bestehenden Probandenkollektivs im Alter von 3 bis 12 Jahren (14 männlich, 11 weiblich), welche gleichermaßen an akuten Rhinitiden litten, mit den für die Kindertherapie bestimmten Rezepturen 3 bzw. 4 bzw. 7 bzw. 8 bzw. 10 für die Dauer der Erkrankung (im Allgemeinen ebenfalls 3 bis 10 Tage) behandelt.

Weitergehende klinischen Untersuchungen sowie weiterführende randomisierte placebokontrollierte Doppelblindstudien der Anmelderin bestätigen darüber hinaus einen Synergismus der Wirkungen der Wirkstoffe der erfindungsgemäßen Zusammensetzungen, was bei der Behandlung von Rhinitiden zu einer klinisch deutlicheren Besserung führt, und zwar über das Ausmaß der Einzelwirkstoffe deutlich hinausgehend.

### 3. Stabilitätsuntersuchungen:

Nachfolgend sind die Ergebnisse einer Vielzahl von Stabilitätsuntersuchungen wiedergegeben.

Zu diesem Zweck wurden jeweils unter definierten Lagerbedingungen, wie sie nachstehend jeweils angegeben sind, Langzeitstabilitätsuntersuchungen an den betreffenden Zusammensetzungen durchgeführt, wobei nach definierten Zeiträumen spezielle Prüfparameter an den Zusammensetzungen gemessen und mit der betreffenden Spezifikation verglichen wurden (z. B. Aussehen der Zusammensetzung, pH-Wert, relative Dichte, Osmolalität sowie Gehalt an Wirkstoffen sowie an Verunreinigungen). Sofern nachstehend nicht ausdrücklich anders angegeben, erfolgte die Lagerung bei einer definierten Temperatur von 25 °C ± 2 °C und bei einer relativen Luftfeuchte der Umgebung von 60 % ± 5 %.

Ein charakteristisches Maß für die Stabilität einer Zusammensetzung ist zum einen die Konstanz des pH-Wertes sowie die Konstanz des Gehalts an Wirkstoffen (d. h. Xylometazolinhydrochlorid bzw. Oxymetazolinhydrochlorid sowie Dexpanthenol). Weiterhin kann der Gehalt an Abbauprodukten der Wirkstoffe bestimmt werden, um die Stabilität der Zusammensetzung zu bewerten.

Wie eingangs geschildert, ist die sogenannte Verunreinigung A ein typisches Abbauprodukt des Xylometazolinhydrochlorids, während als Abbauprodukte des Dexpanthenols Aminopropanol bzw. dessen Ammoniumsalz und D-Pantolacton charakteristisch sind. In der nachfolgenden Beschreibung der Ergebnisse der Stabilitätsuntersuchungen beziehen sich die Prozentangaben der Wirkstoffe Xylometazolinhydrochlorid bzw. Oxymetazolinhydrochlorid und Dexpanthenol auf Gewichtsprozentangaben mit Bezug auf die Zusammensetzung, während dagegen die prozentualen Gehaltsangaben aller Verunreinigungen jeweils als Gewichtsprozentangaben mit Bezug auf den ursprünglichen Wirkstoff bezogen sind, d. h. beispielsweise der Gehalt an Verunreinigung A als Gewichtsprozentangabe in Bezug auf Xylometazolinhydrochlorid und der Gehalt an Aminopropanol bzw. D-Pantolacton als Gewichtsprozentangabe in Bezug auf Dexpanthenol.

Mit anderen Worten beziehen sich bei den Verunreinigungen die Gewichtsprozentangaben gerade nicht auf die Zusammensetzung, sondern auf den Gehalt des betreffenden Wirkstoffs, aus dessen Abbau die betreffende Verunreinigung resultiert.

### a) Stabilitätsuntersuchungen

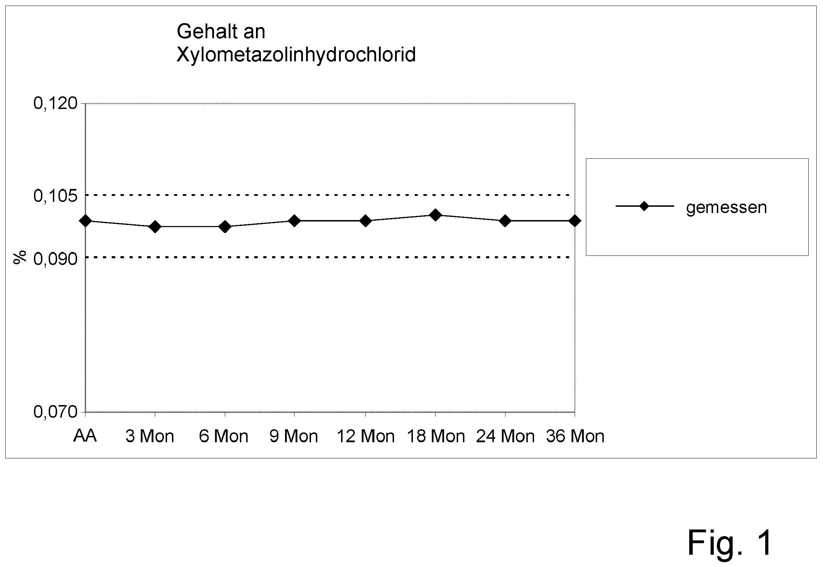

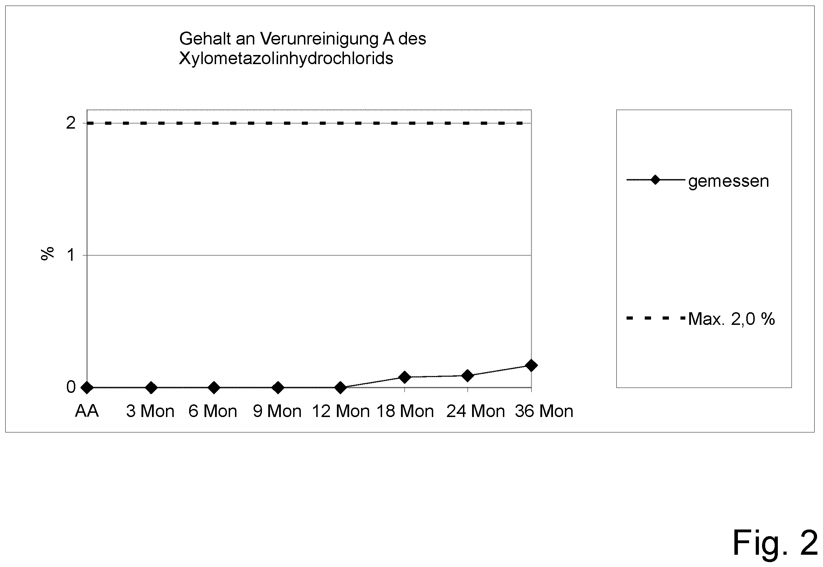

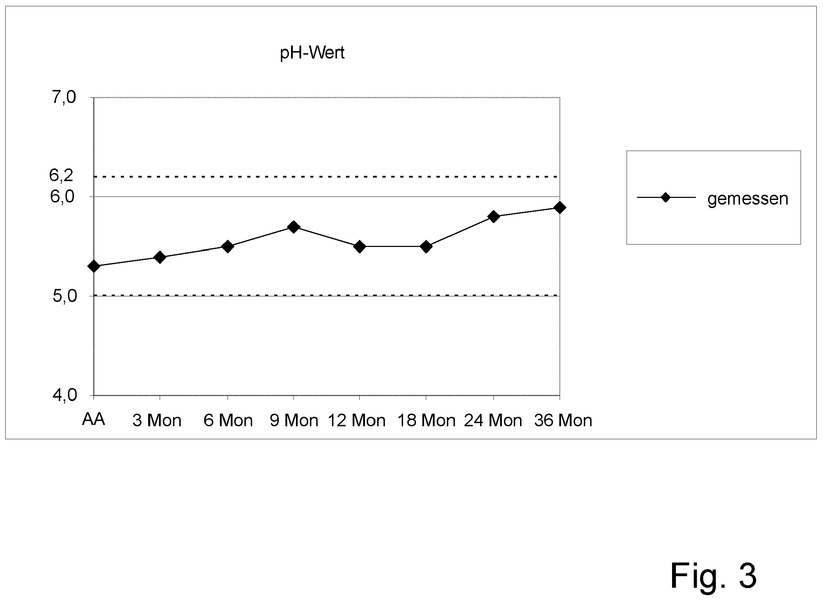

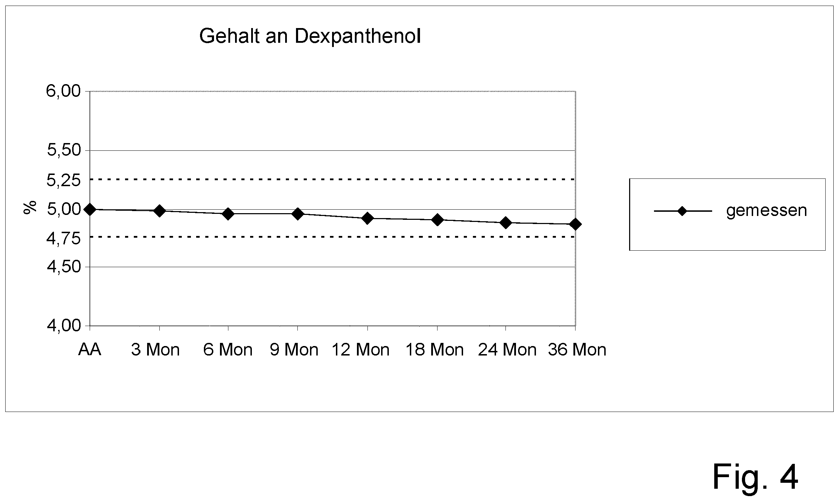

An einer Charge einer erfindungsgemäßen Zusammensetzung gemäß der zuvor beschriebenen Rezeptur 1 wurden Stabilitätsuntersuchungen durchgeführt. Zu diesem Zweck wurde zum einen sowohl der Gehalt an Xylometazolinhydrochlorid (vgl. Fig. 1) als auch der Gehalt an dessen Abbauprodukt (Verunreinigung A) (vgl. Fig. 2) bestimmt, und zwar jeweils nach den in Fig. 1 und 2 angegebenen Lagerzeiten von 3 Monaten, 6 Monaten, 9 Monaten, 12 Monaten, 18 Monaten, 24 Monaten und 36 Monaten. Zum anderen wurde über denselben Lagerzeitraum für die vorgenannten Lagerzeiten auch der pH-Wert (vgl. Fig. 3) als auch der Gehalt an Dexpanthenol (vgl. Fig. 4) bestimmt. Die Lagerung erfolgte bei einer definierten Temperatur von 25 °C ± 2 °C und bei einer relativen Luftfeuchte der Umgebung von 60 % ± 5 %. Die erhaltenen Ergebnisse sind in den Figurendarstellungen gemäß Fig. 1 bis 4 dargestellt.

Fig. 1 zeigt den relativen Gehalt an Xylometazolin in Gewichtsprozent, bezogen auf die Zusammensetzung, über den Lagerzeitraum von 36 Monaten. Es wird eine ausgezeichnete Stabilität des Xylometazolinhydrochlorids beobachtet, d. h. es erfolgt im Wesentlichen kein Abbau.

Dies korreliert mit den Ergebnissen, wie sie in Fig. 2 dargestellt sind, wobei Fig. 2 den relativen Gehalt an Verunreinigungen A in Gewichtsprozent, bezogen auf das Xylometazolinhydrochlorid, über die Lagerzeit darstellt. Über die ersten 12 Monate Lagerzeit wird keinerlei Gehalt an Verunreinigung A festgestellt. Auch in den verbleibenden 24 Monaten steigt der Gehalt an Verunreinigung A nicht signifikant an und liegt weit unterhalb der tolerierbaren Spezifikationsgrenze von 2 Gew.-%, bezogen auf Xylometazolinhydrochlorid.

Wie Fig. 3 weiterhin zeigt, bleibt auch der pH-Wert der erfindungsgemäßen Zusammensetzung für den gesamten Lagerzeitraum von 36 Monaten deutlich innerhalb der Spezifikationsgrenzen zwischen 5,0 und 6,2.

Schließlich belegt auch Fig. 4 die Stabilität der erfindungsgemäßen Zusammensetzung, reflektiert am Gehalt an Dexpanthenol in der erfindungsgemäßen Zusammensetzung in Gewichtsprozent, bezogen auf die Zusammensetzung, wobei der betreffende Gehalt des Dexpanthenols geringfügig abnimmt über die gesamte Lagerzeit von 36 Monaten, aber immer noch deutlich innerhalb der Spezifikationsgrenzen zwischen 4,75 Gew.-% und 5,25 Gew.-%, bezogen auf die Zusammensetzung, bleibt.

Die Ergebnisse belegen die ausgezeichnete Langzeitstabilität der erfindungsgemäßen Zusammensetzung

### b) Weitere Stabilitätsuntersuchungen

An einer weiteren Charge einer erfindungsgemäßen Zusammensetzung gemäß Rezeptur 1 wurde ebenfalls eine Langzeitstabilitätsuntersuchung durchgeführt. Die Ergebnisse sind in der Tabelle 1 wiedergegeben.

### c) Einfluss verschiedener Parameter auf die Stabilität der Zusammensetzung

### Einfluss des pH-Werts

Es wurden verschiedene Zusammensetzungen analog der Rezeptur 1 hergestellt, wobei jedoch in Abweichung von Rezeptur 1 der pH-Wert im Bereich von 4,5 bis 7,5 variiert wurde. Die Ergebnisse sind in Tabelle 2 zusammengefasst. Wie die Ergebnisse zeigen, tritt unterhalb eines pH-Werts von 5,0 und oberhalb eines pH-Werts von 6,2 eine rasche Zersetzung der erfindungsgemäßen Zusammensetzung ein. Im erfindungsgemäß vorgesehenen pH-Bereich zwischen 5,0 und 6,2 werden dagegen gute Ergebnisse erhalten. Besonders gute Ergebnisse lassen sich im pH-Bereich von 5,0 bis 6,0, insbesondere 5,1 bis 6,0, besonders 5,2 bis 5,9, erhalten.

### Einfluss der Art des Puffersystems

Weiterhin wurden Untersuchungen mit unterschiedlichen Puffersystemen durchgeführt. Neben dem erfindungsgemäß eingesetzten Phosphatpuffersystem wurden auch Puffersysteme auf Basis von Kohlensäure/Bicarbonat, Essigsäure/Acetat, Kohlensäure/Silikat und Citronensäure/Citrat untersucht. Der Anfangs-pH-Wert aller Zusammensetzungen wurde auf ca. 5,5 eingestellt. Nur mit dem erfindungsgemäß verwendeten Phosphatpuffersystem lässt sich eine zuverlässige Langzeitstabilität erreichen. Die Ergebnisse sind in Tabelle 3 dargestellt.

### Einfluss der Anwesenheit von Konservierungs- bzw. Desinfektionsmitteln

Weiterhin wurde der Einfluss von Konservierungs- bzw. Desinfektionsmitteln in Bezug auf die Stabilität der Zusammensetzung untersucht. Wie die Untersuchungen zeigen, wird durch die Anwesenheit eines Konservierungs- bzw. Desinfektionsmittels (Benzalkoniumchlorid) die Langzeitstabilität der Zusammensetzung noch weiterführend verbessert. Die betreffenden Ergebnisse sind in der Tabelle 4 wiedergegeben.

### Einfluss des Dihydrogenphosphat/Monohydrogenposphat-Molverhältnisses

Weiterhin wurde der Einfluss des Dihydrogenphosphat/Monohydrogenposphat-Molverhältnisses untersucht. Nur mit dem erfindungsgemäß gewählten Molverhältnis lässt sich eine besonders effiziente Langzeitstabilisierung der Zusammensetzung erhalten.

Wie die vorstehend referierten Ergebnisse der Stabilitätsuntersuchungen der Anmelderin belegen, bedurfte es zur Auffindung einer stabilen Zusammensetzung für die Behandlung von Rhinitiden nach Art der vorliegenden Erfindung eines intensiven Forschungsaufwands seitens der Anmelderin.

Die vorstehend referierten Stabilitätsuntersuchungen zeigen die Komplexität des von der Anmelderin aufgefundenen Lösungsansatzes. Hierzu zählen die gemeinsame Anwesenheit der Wirkstoffe a) und b) in einem definierten Mengenverhältnis unter gleichzeitiger Einhaltung bzw. Konstanthaltung des pH-Werts der Zusammensetzung im zuvor definierten Bereich - ebenso wie weitere Cofaktoren, wie insbesondere die Auswahl des geeigneten Puffersystems sowie des geeigneten Molverhältnisses der Komponenten dieses Puffersystems. Eine zusätzliche Stabilitätsverbesserung lässt sich durch Konservierungs- und/oder Desinfektionsmittel, insbesondere auf Basis von Benzalkoniumchlorid, erreichen.

Der erfindungsgemäße Lösungsansatz kommt im Ergebnis in überraschender Weise mit nur wenigen zusätzlichen Inhaltsstoffen aus, welche aber die Wirkung der eigentlichen Wirkstoffe nicht beeinträchtigen; dies ist im Hinblick auf die beabsichtigte pharmazeutische Wirkung von entscheidender Bedeutung.

**Tabelle 1**

| **Prüfparameter** | **Spezifikation** | **Anfang** | **3 Monate** | **6 Monate** | **9 Monate** | **12 Monate** | **18 Monate** | **24 Monate** | **36 Monate** |
|---|---|---|---|---|---|---|---|---|---|
| **Aussehen** | farblose klare Flüssigkeit (Klarheit: < Ref.-Susp.; Färbung: < B9) | erfüllt | erfüllt | erfüllt | erfüllt | erfüllt | erfüllt | erfüllt | erfüllt |
| **Relat. Dichte (20 °C)** | 1,005-1,105 | 1,0153 | 1,0153 | 1,0153 | 1,0153 | 1,0154 | 1,0149 | 1,0155 | 1,0153 |
| **Osmolalität (mosm/kg)** | 350-495 | 413 | 420 | 414 | 417 | 425 | 429 | 427 | 430 |
| **Xylometazolinhydrochlorid (Gew.-%)** | 0,100 (0,095-0,105) | 0,1013 | 0,1036 | 0,1028 | 0,1020 | 0,1032 | 0,1017 | 0,1044 | 0,1015 |
| **Verunreinigung A (Gew.-%)** | max. 1,0 | n.b. | n.b. | 0,013 | 0,068 | 0,070 | 0,075 | 0,092 | 0,160 |
| **Dexpanthenol (Gew.-%)** | 5,00 (4,50-5,25) | 5,21 | 5,24 | 5,24 | 5,15 | 5,25 | 5,17 | 5,19 | 5,15 |
| **3-Aminopropanol (Gew.-%)** | max. 3,0 | 0,32 | 0,51 | 0,67 | 0,76 | 0,83 | 0,90 | 0,94 | 0,99 |
| **D-Pantolacton (Gew.-%)** | max. 3,0 | n.b. | n.b. | 0,0100 | 0,0186 | 0,0210 | 0,0330 | 0,0450 | 0,0600 |
| **Sterilität** | Ph.Eur. 5.1.4 2 Kat. 1 | erfüllt | erfüllt | erfüllt | erfüllt | erfüllt | erfüllt | erfüllt | erfüllt |

| | | | | | | | | | |
|---|---|---|---|---|---|---|---|---|---|
| n.b. = nicht bestimmbar (unterhalb der Analysegrenze) | | | | | | | | | |

**Tabelle 2**

| **Lfd. Nr.** | **Prüfparameter** | **Spezifikation** | **Anfang** | **3 Monate** | **6 Monate** | **9 Monate** | **12 Monate** | **18 Monate** | **24 Monate** |
|---|---|---|---|---|---|---|---|---|---|
| **2A*** | **Xylometazolinhydrochlorid (Gew.-%)** | 0,100 (0,095-0,105) | 0,1011 | 0,1000 | 0,0998 | 0,0980 | 0,0945 | 0,0890 | 0,0802 |
| | **Verunreinig. A (Gew.- %)** | max. 1,0 | 0,071 | 0,441 | > 1 | > 1 | > 1 | > 1 | > 1 |
| | **Dexpanthenol (Gew.- %)** | 5,00(4,50-5,25) | 5,19 | 5,01 | 4,83 | 4,69 | < 4,50 | < 4,50 | < 4,50 |
| | **3-Aminopropanol(Gew.-%)** | max. 3,0 | 0,41 | 1,12 | > 3 | > 3 | > 3 | > 3 | > 3 |
| **2B** | **Xylometazolinhyd rochlorid (Gew.-%)** | 0,100 (0,095-0,105) | 0,1013 | 0,1029 | 0,1021 | 0,1022 | 0,1030 | 0,1019 | 0,1033 |
| | **Verunreinig. A (Gew.-%)** | max. 1,0 | n.b. | n.b. | 0,019 | 0,070 | 0,079 | 0,089 | 0,090 |
| | **Dexpanthenol (Gew.-%)** | 5,00(4,50-5,25) | 5,21 | 5,20 | 5,23 | 5,19 | 5,24 | 5,13 | 5,10 |
| | **3-Aminopropanol(Gew.-%)** | max. 3,0 | 0,35 | 0,45 | 0,67 | 0,81 | 0,93 | 1,02 | 1,12 |
| **2C** | **Xylometazolinhydrochlorid (Gew.-%)** | 0,100 (0,095-0,105) | 0,1013 | 0,1036 | 0,1028 | 0,1020 | 0,1032 | 0,1017 | 0,1044 |
| | **Verunreinig. A (Gew.-%)** | max. 1,0 | n.b. | n.b. | n.b. | 0,068 | 0,070 | 0,075 | 0,092 |
| | **Dexpanthenol (Gew.-%)** | 5,00(4,50-5,25) | 5,21 | 5,24 | 5,24 | 5,15 | 5,25 | 5,17 | 5,19 |
| | **3-Aminopropanol(Gew.- %)** | max. 3,0 | 0,29 | 0,37 | 0,52 | 0,65 | 0,75 | 0,82 | 0,99 |

| | | | | | | | | | |
|---|---|---|---|---|---|---|---|---|---|
| n.b. = nicht bestimmbar (unterhalb der Analysegrenze) * = nicht erfindungsgemäß | | | | | | | | | |

**Tabelle 2 (Fortsetzung)**

| **Lfd. Nr.** | **Prüfparameter** | **Spezifikation** | **Anfang** | **3 Monate** | **6 Monate** | **9 Monate** | **12 Monate** | **18 Monate** | **24 Monate** |
|---|---|---|---|---|---|---|---|---|---|
| **2D** | **Xylometazolinhydrochlorid (Gew.-%)** | 0,100 (0,095-0,105) | 0,1013 | 0,1016 | 0,1018 | 0,1022 | 0,1033 | 0,1019 | 0,1010 |
| | **Verunreinig. A (Gew.- %)** | max. 1,0 | n.b. | n.b. | n.b. | n.b. | 0,055 | 0,061 | 0,071 |
| | **Dexpanthenol (Gew.-%)** | 5,00(4,50-5,25) | 5,21 | 5,21 | 5,21 | 5,20 | 5,24 | 5,20 | 5,19 |
| | **3-Aminopropanol(Gew.-%)** | max. 3,0 | 0,31 | 0,43 | 0,51 | 0,63 | 0,72 | 0,88 | 0,98 |
| **2E*** | **Xylometazolinhydrochlorid (Gew.-%)** | 0,100 (0,095-0,105) | 0,1011 | 0,1000 | 0,1018 | 0,0999 | 0,0955 | 0,0901 | 0,0802 |
| | **Verunreinig. A (Gew.-%)** | max. 1,0 | 0,056 | 0,260 | 0,988 | > 1 | > 1 | > 1 | > 1 |
| | **Dexpanthenol (Gew.- %)** | 5,00(4,50-5,25) | 5,21 | 5,01 | 4,90 | 4,80 | 4,70 | < 4,50 | < 4,50 |
| | **3-Aminopropanol(Gew.-%)** | max. 3,0 | 0,15 | 0,99 | 1,90 | > 3 | > 3 | > 3 | > 3 |
| **2F*** | **Xylometazolinhydrochlorid (Gew.-%)** | 0,100 (0,095-0,105) | 0,1010 | 0,1000 | 0,1018 | 0,0999 | 0,0955 | 0,0901 | 0,0802 |
| | **Verunreinig. A (Gew.- %)** | max. 1,0 | 0,075 | 0,489 | > 1 | > 1 | > 1 | > 1 | > 1 |
| | **Dexpanthenol (Gew.- %)** | 5,00(4,50-5,25) | 5,20 | 4,99 | 4,80 | 4,60 | < 4,50 | < 4,50 | < 4,50 |
| | **3-Aminopropanol(Gew.-%)** | max. 3,0 | 0,22 | 1,20 | > 3 | > 3 | > 3 | > 3 | > 3 |

| | | | | | | | | | |
|---|---|---|---|---|---|---|---|---|---|
| n.b. = nicht bestimmbar (unterhalb der Analysegrenze) * = nicht erfindungsgemäß Anfangs-pH-Wert Zusammens.2A: 4,0 / Anfangs-pH-Wert Zusammens.2B: 5,0 / Anfangs-pH-Wert Zusammens.2C: 5,2 Anfangs-pH-Wert Zusammens.2D: 5,5 / Anfangs-pH-Wert Zusammens.2E: 6,5 / Anfangs-pH-Wert Zusammens.2F: 7,5 / | | | | | | | | | |

**Tabelle 3**

| **Lfd. Nr.** | **Prüfparameter** | **Spezifikation** | **Anfang** | **3 Monate** | **6 Monate** | **9 Monate** | **12 Monate** | **18 Monate** | **24 Monate** |
|---|---|---|---|---|---|---|---|---|---|
| **3A*** | **Xylometazolinhydrochlorid (Gew.-%)** | 0,100 (0,095-0,105) | 0,1013 | 0,1011 | 0,0997 | 0,0988 | 0,0942 | 0,0890 | 0,0800 |
| | **Verunreinig. A (Gew.- %)** | max. 1,0 | 0,039 | 0,342 | 0,663 | 0,897 | > 1 | >1 | > 1 |
| | **Dexpanthenol (Gew.- %)** | 5,00(4,50-5,25) | 5,21 | 5,10 | 5,00 | 4,86 | 4,57 | < 4,50 | < 4,50 |
| | **3-Aminopropanol(Gew.-%)** | max. 3,0 | 0,19 | 1,29 | 2,30 | 2,99 | > 3 | > 3 | > 3 |
| **3B*** | **Xylometazolinhydrochlorid (Gew.-%)** | 0,100 (0,095-0,105) | 0,1013 | 0,1012 | 0,1010 | 0,1003 | 0,0995 | 0,0978 | 0,0922 |
| | **Verunreinig. A (Gew.-%)** | max. 1,0 | n.b. | 0,220 | 0,362 | 0,443 | 0,677 | > 1 | > 1 |
| | **Dexpanthenol (Gew.-%)** | 5,00(4,50-5,25) | 5,21 | 5,20 | 5,08 | 4,98 | 4,67 | < 4,50 | < 4,50 |
| | **3-Aminopropanol(Gew.-%)** | max. 3,0 | n.b. | 1,11 | 1,70 | 2,99 | > 3 | > 3 | > 3 |

| | | | | | | | | | |
|---|---|---|---|---|---|---|---|---|---|
| n.b. = nicht bestimmbar (unterhalb der Analysegrenze) * = nicht erfindungsgemäß | | | | | | | | | |

**Tabelle 3 (Fortsetzung)**

| **Lfd. Nr.** | **Prüfparameter** | **Spezifikation** | **Anfang** | **3 Monate** | **6 Monate** | **9 Monate** | **12 Monate** | **18 Monate** | **24 Monate** |
|---|---|---|---|---|---|---|---|---|---|
| **3C** | **Xylometazolinhydrochlorid (Gew.-%)** | 0,100 (0,095-0,105) | 0,1013 | 0,1016 | 0,1018 | 0,1022 | 0,1033 | 0,1019 | 0,1010 |
| | **Verunreinig. A (Gew.-%)** | max. 1,0 | n.b. | n.b. | n.b. | n.b. | 0,055 | 0,061 | 0,071 |
| | **Dexpanthenol (Gew.-%)** | 5,00(4,50-5,25) | 5,21 | 5,21 | 5,21 | 5,20 | 5,24 | 5,20 | 5,19 |
| | **3-Aminopropanol(Gew.-%)** | max. 3,0 | 0,33 | 0,41 | 0,52 | 0,63 | 0,77 | 0,87 | 0,95 |
| **3D*** | **Xylometazolinhydrochlorid (Gew.-%)** | 0,100 (0,095-0,105) | 0,1011 | 0,1009 | 0,1000 | 0,0999 | 0,0955 | 0,0940 | 0,0902 |
| | **Verunreinig. A (Gew.-%)** | max. 1,0 | 0,046 | 0,255 | 0,560 | 0,789 | 0,999 | > 1 | > 1 |
| | **Dexpanthenol (Gew.- %)** | 5,00(4,50-5,25) | 5,22 | 5,11 | 5,01 | 4,99 | 4,67 | < 4,50 | < 4,50 |
| | **3-Aminopropanol(Gew.-%)** | max. 3,0 | 0,15 | 0,99 | 1,90 | 2,90 | > 3 | > 3 | > 3 |
| **3E*** | **Xylometazolinhydrochlorid (Gew.-%)** | 0,100 (0,095-0,105) | 0,1012 | 0,1014 | 0,1011 | 0,1001 | 0,0990 | 0,0980 | 0,0902 |
| | **Verunreinig. A (Gew.-%)** | max. 1,0 | n.b. | 0,190 | 0,370 | 0,489 | 0,789 | 0,999 | > 1 |
| | **Dexpanthenol (Gew.- %)** | 5,00(4,50-5,25) | 5,21 | 5,19 | 5,11 | 5,00 | 4,78 | < 4,50 | < 4,50 |
| | **3-Aminopropanol(Gew.-%)** | max. 3,0 | n.b. | 0,99 | 1,90 | 2,90 | > 3 | > 3 | > 3 |

| | | | | | | | | | |
|---|---|---|---|---|---|---|---|---|---|
| n.b. = nicht bestimmbar (unterhalb der Analysegrenze) * = nicht erfindungsgemäß Puffer: 3A: Kohlensäure/Bicarbonat / 3B: Essigsäure/Acetat / 3C: Phosphatpuffer (Erfindung) 3D: Kohlensäure/Silikat / 3E:Citronensäure/Citrat | | | | | | | | | |

**Tabelle 4**

| **Lfd. Nr.** | **Prüfparameter** | **Spezifikation** | **Anfang** | **3 Monate** | **6 Monate** | **9 Monate** | **12 Monate** | **18 Monate** | **24 Monate** |
|---|---|---|---|---|---|---|---|---|---|
| **4A** | **Xylometazolinhydrochlorid (Gew.-%)** | 0,100 (0,095-0,105) | 0,1013 | 0,1016 | 0,1018 | 0,1022 | 0,1033 | 0,1019 | 0,1010 |
| | **Verunreinig. A (Gew.- %)** | max. 1,0 | n.b. | n.b. | n.b. | n.b. | 0,055 | 0,061 | 0,071 |
| | **Dexpanthenol (Gew.-%)** | 5,00(4,50-5,25) | 5,21 | 5,21 | 5,21 | 5,20 | 5,24 | 5,20 | 5,19 |
| | **3-Aminopropanol(Gew.-%)** | max. 3,0 | 0,33 | 0,43 | 0,52 | 0,66 | 0,73 | 0,87 | 0,98 |
| **4B** | **Xylometazolinhydrochlorid (Gew.-%)** | 0,100 (0,095-0,105) | 0,1013 | 0,1016 | 0,1016 | 0,1020 | 0,1029 | 0,1020 | 0,1015 |
| | **Verunreinig. A (Gew.-%)** | max. 1,0 | n.b. | n.b. | n.b. | n.b. | n.b. | 0,033 | 0,049 |
| | **Dexpanthenol (Gew.- %)** | 5,00(4,50-5,25) | 5,21 | 5,21 | 5,21 | 5,20 | 5,20 | 5,20 | 5,20 |
| | **3-Aminopropanol(Gew.-%)** | max. 3,0 | 0,32 | 0,42 | 0,51 | 0,64 | 0,73 | 0,86 | 0,97 |

| | | | | | | | | | |
|---|---|---|---|---|---|---|---|---|---|
| Zusammensetzung 4A: ohne Benzalkoniumchlorid Zusammensetzung 4B: wie Zusammensetzung 4A, jedoch mit Benzalkoniumchlorid (0,02 Gew.-%) | | | | | | | | | |

## Patentansprüche

1. Zusammensetzung, insbesondere pharmazeutische Zusammensetzung, vorzugsweise für die topische, insbesondere nasale, bevorzugt intranasale Applikation, insbesondere für die Behandlung von Rhinitiden,
wobei die Zusammensetzung
a) mindestens ein imidazolinbasiertes alpha-Sympathomimetikum oder dessen physiologisch unbedenkliches Salz; und
b)
(b1) Pantothenol (Dexpanthenol) oder dessen physiologisch unbedenkliche Ester und/oder
(b2) Pantothensäure oder deren physiologisch unbedenkliche Salze
enthält,
**dadurch gekennzeichnet,**
**dass** der pH-Wert der Zusammensetzung im Bereich von 5,0 bis 6,2 eingestellt und/oder konstantgehalten ist.

2. Zusammensetzung nach Anspruch 1, **dadurch gekennzeichnet,**
**dass** die Komponente a) und/oder das imidazolinbasierte alpha-Sympathomimetikum ausgewählt ist aus Xylometazolin oder Oxymetazolin, insbesondere in Form von deren physiologisch unbedenklichen Salzen, besonders bevorzugt in Form von deren Hydrochloridsalzen; und/oder
**dass** die Komponente a) und/oder das imidazolinbasierte alpha-Sympathomimetikum ausgewählt ist aus Xylometazolin, insbesondere in Form von dessen physiologisch unbedenklichen Salzen, besonders bevorzugt in Form von dessen Hydrochloridsalz (Xylometazolinhydrochlorid); und/oder
**dass** die Komponente b) aus Pantothenol (Dexpanthenol) oder dessen physiologisch unbedenklichen Estern ausgewählt ist und vorzugsweise Pantothenol (Dexpanthenol) ist.

3. Zusammensetzung nach Anspruch 1 oder 2, **dadurch gekennzeichnet, dass** die Zusammensetzung
a) mindestens ein imidazolinbasiertes alpha-Sympathomimetikum aus der Gruppe von Xylometazolin und/oder Oxymetazolin oder deren physiologisch unbedenklichen Salzen, besonders bevorzugt in Form von deren Hydrochloridsalzen; und
b) Pantothenol (Dexpanthenol) oder dessen physiologisch unbedenkliche Ester
enthält; und/oder
dass die Zusammensetzung
a) Xylometazolin oder deren physiologisch unbedenkliche Salze, besonders bevorzugt in Form von dessen Hydrochloridsalz (Xylometazolinhydrochlorid);
und
b) Pantothenol (Dexpanthenol) oder dessen physiologisch unbedenkliche Ester, vorzugsweise Pantothenol (Dexpanthenol), enthält; und/oder
dass die Zusammensetzung die Komponente a), bezogen auf die Zusammensetzung, in einer Menge von 0,001 bis 2 Gew.-%, insbesondere 0,005 bis 1,5 Gew.-%, vorzugsweise 0,01 bis 1,2 Gew.-%, bevorzugt 0,02 bis 1,0 Gew.-%, besonders bevorzugt 0,03 bis 0,5 Gew.-%, ganz besonders bevorzugt 0,04 bis 0,2 Gew.-%, enthält; und/oder
dass die Zusammensetzung die Komponente b), bezogen auf die Zusammensetzung, in einer Menge von 0,01 bis 10 Gew.-%, insbesondere 0,1 bis 9 Gew.-%, vorzugsweise 0,5 bis 8 Gew.-%, bevorzugt 1 bis 7 Gew.-%, besonders bevorzugt 2 bis 6 Gew.-%, ganz besonders bevorzugt 3 bis 6 Gew.-%, enthält; und/oder
dass die Zusammensetzung die Komponenten a) und b) in einem Mengenverhältnis von Komponente a) zu Komponente b) im Bereich von 1 : 10 bis 1 : 1.000, insbesondere 1 : 15 bis 1 : 500, vorzugsweise 1 : 20 bis 1 : 250, bevorzugt 1 : 25 bis 1 : 200, besonders bevorzugt 1 :30 bis 1 : 175, ganz besonders bevorzugt 1 : 40 bis 1 : 150, noch mehr bevorzugt 1 : 45 bis 1 : 125, enthält.

4. Zusammensetzung nach einem der vorangehenden Ansprüche, **dadurch gekennzeichnet,**
**dass** die Zusammensetzung als wässrige Zusammensetzung vorliegt und/oder dass die Zusammensetzung wässrig basiert ist und/oder wässrig formuliert vorliegt, insbesondere in Form einer wässrigen Lösung oder wässrigen Solubilisierung; und/oder
**dass** die Zusammensetzung als wässriges System, insbesondere als wässriges Einphasensystem, vorzugsweise als wässrige Lösung oder wässrige Solubilisierung, vorliegt und/oder dass die Zusammensetzung einen Exzipienten oder Träger auf Wasserbasis aufweist und/oder dass die Zusammensetzung als klare, farblose wässrige Lösung vorliegt; und/oder dass der pH-Wert der Zusammensetzung im Bereich von 5,0 bis 6,0, insbesondere im Bereich von 5,1 bis 6,0, vorzugsweise im Bereich von 5,2 bis 5,9, eingestellt und/oder konstantgehalten wird.

5. Zusammensetzung nach einem der vorangehenden Ansprüche, **dadurch gekennzeichnet,**
**dass** die Einstellung und/oder Konstanthaltung des pH-Werts der Zusammensetzung mittels mindestens eines chemischen Puffersystems, insbesondere Puffersalz(en), erfolgt, insbesondere wobei als chemisches Puffersystem bevorzugt ein Dihydrogenphosphat/Monohydrogenposphat-Puffersystem ("H₂PO₄⁻/HPO₄²⁻-Puffer(system)" bzw. "Phosphatpuffer-(system)"), insbesondere ein Alkalidihydrogenphosphat/Alkalimonohydrogenposphat-Puffersystem, eingesetzt wird, und/oder dass die Zusammensetzung mindestens ein chemisches Puffersystem, insbesondere Puffersalz(e), enthält, insbesondere wobei die Zusammensetzung als chemisches Puffersystem bevorzugt ein Dihydrogenphosphat/Monohydrogenposphat-Puffersystem ("H₂PO₄⁻/HPO₄⁻²-Puffer(system)" bzw. "Phosphatpuffer(system)"), insbesondere ein Alkalidihydrogenphosphat/Alkalimonohydrogenposphat-Puffersystem, enthält;
insbesondere wobei das Dihydrogenphosphat/Monohydrogenposphat-Puffersystem mit einem Dihydrogenphosphat/Monohydrogenposphat-Molverhältnis von größer 5 : 1, insbesondere im Bereich von 5 : 1 bis 110 : 1, besonders bevorzugt im Bereich von 6 : 1 bis 105 : 1, ganz besonders bevorzugt im Bereich von 7 : 1 bis 100 : 1, eingesetzt wird und/oder insbesondere wobei die Zusammensetzung das Dihydrogenphosphat/Monohydrogenposphat-Puffersystem mit einem Dihydrogenphosphat/Monohydrogenposphat-Molverhältnis von größer 5 : 1, insbesondere im Bereich von 5 : 1 bis 110 : 1, besonders bevorzugt im Bereich von 6 : 1 bis 105 : 1, ganz besonders bevorzugt im Bereich von 7 : 1 bis 100 : 1, enthält.

6. Zusammensetzung nach einem der vorangehenden Ansprüche, **dadurch gekennzeichnet, dass**
die Zusammensetzung c) mindestens ein Konservierungsmittel und/oder Desinfektionsmittel enthält, insbesondere wobei das Konservierungsmittel und/oder Desinfektionsmittel ausgewählt ist aus der Gruppe von (i) Alkylbenzyldimethylammoniumchloriden, insbesondere C₈-C₁₈-Alkylbenzyldimethylammoniumchloriden, und Mischungen von verschiedenen Alkylbenzyldimethylammoniumchloriden, vorzugsweise Benzalkoniumchlorid, (ii) para-Hydroxybenzoesäureestern und Mischungen von verschiedenen para-Hydroxybenzoesäureestern, vorzugsweise Nipa-Estern, (iii) Chlorhexidin sowie Kombinationen der vorgenannten Verbindungen und besonders bevorzugt Benzalkoniumchlorid ist und/oder insbesondere wobei die Zusammensetzung das Konservierungsmittel und/oder Desinfektionsmittel, bezogen auf die Zusammensetzung, in einer Menge von 0,001 bis 10 Gew.-%, insbesondere 0,005 bis 5 Gew.-%, vorzugsweise 0,01 bis 2 Gew.-%, bevorzugt 0,01 bis 1 Gew.-%, besonders bevorzugt 0,01 bis 0,5 Gew.-%, enthält; und/oder dass die Zusammensetzung d) mindestens ein vorzugsweise saures Glykosaminoglykan oder dessen physiologisch unbedenkliche Salze oder Derivate, insbesondere Hyaluronsäure oder deren physiologisch unbedenkliche Salze, enthält, insbesondere in einer Menge von 0,0001 bis 10 Gew.-%, insbesondere 0,001 bis 5 Gew.-%, vorzugsweise 0,01 bis 2 Gew.-%, bezogen auf die Zusammensetzung; und/oder
dass die Zusammensetzung e) Ectoin oder mindestens ein Ectoinderivat, insbesondere ein Hydroxyectoin, enthält, insbesondere in einer Menge von 0,0001 bis 10 Gew.-%, insbesondere 0,001 bis 5 Gew.-%, vorzugsweise 0,01 bis 2 Gew.-%, bezogen auf die Zusammensetzung; und/oder dass die Zusammensetzung f) Natriumchlorid enthält, insbesondere in einer Menge von 0,001 bis 5 Gew.-%, insbesondere 0,01 bis 2 Gew.-%, vorzugsweise 0,1 bis 1 Gew.-%, bezogen auf die Zusammensetzung; und/oder
dass die Zusammensetzung mindestens einen weiteren Inhaltsstoff aufweist, insbesondere ausgewählt aus der Gruppe von Verarbeitungshilfsstoffen, Stabilisatoren, Emulgatoren, Antioxidantien, Feuchthaltemitteln, Verdickungsmitteln, Antiseptika, Farbstoffen, Aromastoffen, Riechstoffen, Duftstoffen, Streckmitteln, Bindemitteln, Netzmitteln, Vitaminen, Spurenelementen, Mineralstoffen, Mikronährstoffen und/oder ätherischen Ölen sowie deren Kombinationen.

7. Zusammensetzung nach einem der vorangehenden Ansprüche, **dadurch gekennzeichnet,**
**dass** die Zusammensetzung eine Osmolalität im Bereich von 300 bis 600 mosm/kg, insbesondere im Bereich von 310 bis 550 mosm/kg, vorzugsweise im Bereich von 300 bis 525 mosm/kg, bevorzugt im Bereich von 325 bis 510 mosm/kg, besonders bevorzugt im Bereich von 350 bis 500 mosm/kg, aufweist; und/oder
**dass** die Zusammensetzung bei einer Temperatur von 20 °C und bei einem Druck von 1.013,25 mbar eine relative Dichte, bezogen auf reines Wasser, im Bereich von 1,001 bis 1,2, insbesondere im Bereich von 1,005 bis 1,15, vorzugsweise im Bereich von 1,005 bis 1,105, aufweist; und/oder
**dass** die Zusammensetzung bei Temperaturen im Bereich von 20 °C bis 50 °C, bei einem Druck von 1.013,25 mbar und bei einer relativen Luftfeuchtigkeit im Bereich von 50 % bis 90 % mindestens 6 Monate, insbesondere mindestens 12 Monate, vorzugsweise mindestens 24 Monate, bevorzugt mindestens 36 Monate, stabil, insbesondere lagerstabil, ist.

8. Zusammensetzung nach einem der vorangehenden Ansprüche, **dadurch gekennzeichnet,**
**dass** die Zusammensetzung einen Gehalt an Abbauprodukt(en) des Wirkstoffs a), insbesondere Verunreinigung A, von höchstens 5 Gew.-%, insbesondere von höchstens 3 Gew.-%, vorzugsweise von höchstens 2 Gew.-%, besonders bevorzugt von höchstens 1 Gew.-%, bezogen auf den Wirkstoff a), enthält, insbesondere auch nach Lagerung der Zusammensetzung bei Temperaturen im Bereich von 20 °C bis 50 °C, bei einem Druck von 1.013,25 mbar und bei einer relativen Luftfeuchtigkeit im Bereich von 50 % bis 90 % von mindestens 6 Monaten, insbesondere mindestens 12 Monaten, vorzugsweise mindestens 24 Monaten, bevorzugt mindestens 36 Monaten, und/oder
**dass** die Zusammensetzung einen Gehalt an Abbauprodukt(en) des Wirkstoffs b), insbesondere Aminopropanol und/oder D-Pantolacton, von jeweils höchstens 5 Gew.-%, insbesondere von jeweils höchstens 3 Gew.-%, vorzugsweise von jeweils höchstens 2 Gew.-%, besonders bevorzugt von jeweils höchstens 1 Gew.-%, bezogen auf den Wirkstoff b), enthält, insbesondere auch nach Lagerung der Zusammensetzung bei Temperaturen im Bereich von 20 °C bis 50 °C, bei einem Druck von 1.013,25 mbar und bei einer relativen Luftfeuchtigkeit im Bereich von 50 % bis 90 % von mindestens 6 Monaten, insbesondere mindestens 12 Monaten, vorzugsweise mindestens 24 Monaten, bevorzugt mindestens 36 Monaten.

9. Zusammensetzung nach einem der vorangehenden Ansprüche zur Verwendung bei der prophylaktischen und/oder kurativen topischen Behandlung von Rhinitiden, insbesondere *Rhinitis acuta.*

10. Applikationsvorrichtung, insbesondere für die topische, insbesondere nasale, vorzugsweise intranasale Applikation, bevorzugt in Form eines Behältnisses mit Tropf- oder Sprüheinrichtung, enthaltend eine Zusammensetzung nach einem der vorangehenden Ansprüche.

11. Applikationsvorrichtung nach Anspruch 10, **dadurch gekennzeichnet,**
**dass** die Applikationsvorrichtung eine Sprüheinrichtung zur gleichförmigen Ausbringung der Zusammensetzung in einer Menge pro Sprühstoß im Bereich von 25 µl bis 300 µl, insbesondere im Bereich von 50 µl bis 200 µl, vorzugsweise im Bereich von 75 µl bis 125 µl, aufweist; und/oder
**dass** die Applikationsvorrichtung ein Vorratsbehälter mit einem Volumen im Bereich von 5 ml bis 100 ml, insbesondere 10 ml bis 50 ml, aufweist.

12. Verwendung einer Zusammensetzung nach einem der vorangehenden Ansprüche zur prophylaktischen und/oder kurativen topischen Behandlung von Rhinitiden, insbesondere *Rhinitis acuta,* und/oder Verwendung einer Zusammensetzung nach einem der vorangehenden Ansprüche zur Herstellung eines Arzneimittels zur prophylaktischen und/oder kurativen topischen Behandlung von Rhinitiden, insbesondere *Rhinitis acuta.*

13. Verwendung nach Anspruch 12 zur Behandlung von Rhinitiden aller Art, insbesondere *Rhinitis acuta, Rhinitis allergica, Rhinitis atrophicans, Rhinitis hyperplastica* oder *hypertrophicans, Rhinitis mutilans, Rhinitis nervosa* oder *vasomotorica* oder *Rhinitis pseudomembranacea,* vorzugsweise *Rhinitis acuta.*

14. Verwendung nach Anspruch 12 oder 13, **dadurch gekennzeichnet, dass** die pharmazeutische Zusammensetzung nach einem der vorangehenden Ansprüche topisch, insbesondere nasal, vorzugsweise intranasal, appliziert wird.

15. Verfahren zur Stabilisierung eines Wirkstoffes a) und eines Wirkstoffes b) in gemeinsamer wässriger Lösung einer Zusammensetzung, insbesondere pharmazeutischen Zusammensetzung, vorzugsweise für die topische, insbesondere nasale, bevorzugt intranasale Applikation, insbesondere für die Behandlung von Rhinitiden, durch Einstellung und/oder Konstanthalten des pH-Wertes im Bereich von 5,0 bis 6,2,
wobei die Zusammensetzung
a) mindestens ein imidazolinbasiertes alpha-Sympathomimetikum oder dessen physiologisch unbedenkliches Salz; und
b)
(b1) Pantothenol (Dexpanthenol) oder dessen physiologisch unbedenkliche Ester und/oder
(b2) Pantothensäure oder deren physiologisch unbedenkliche Salze
enthält,
wobei der pH-Wert der Zusammensetzung im Bereich von 5,0 bis 6,2 eingestellt und/oder konstantgehalten wird.
